# EUROPEAN PATENT APPLICATION

(11) **EP 4 202 035 A1**
(43) Date of publication of application: **28.06.2023**
(21) Application number: 21216799.3
(22) Date of filing: 22.12.2021
(51) Int. Cl.: C12N 1/00, C12N 15/81, C12P 21/00, C12P 21/02

(54) **IMPROVED EXPRESSION OF PEPTIDES**

(71) Applicant: Gelita AG, 69412 Eberbach (DE)
(72) Inventor: HAUSMANNS, Stephan, 69126 Heidelberg (DE); FRECH, Hans-Ulrich, 69469 Weinheim (DE); HAHN, Martin, 48599 Gronau (DE); OESSER, Steffen, 24960 Glücksburg (DE); BLANK, Lars, 44227 Dortmund (DE); BÜCHS, Jochen, 52064 Aachen (DE); GERMER, Andrea, 52074 Aachen (DE); WOLLBORN, David, 41334 Nettetal (DE)
(74) Representative: Schrell, Andreas

(57) **Abstract**

The present invention relates to fungal cells, in particular methylotrophic fungal cells, for use in improved production of recombinant peptides of interest and processes for producing recombinant peptides of interest.

## Description

The present invention relates to fungal cells, in particular methylotrophic fungal cells, for use in improved production of recombinant peptides of interest and processes for producing recombinant peptides of interest.

The expression of recombinant heterologous proteins and peptides is a challenging process in biotechnology. In particular expressing proteins and peptides originating from eukaryotes in prokaryotic systems can be difficult or even impossible, for instance, due to misfolding. This is often the case for proteins and peptides meant to be secreted as the secretion system differs substantially between eukaryotes and prokaryotes. Eukaryotic proteins and peptides meant for secretion are glycosylated, for which a specific post-translational modification system has evolved in eukaryotes which is lacking in prokaryotes. These post-translational modifications are often required for the applicability of these proteins and peptides or even for their proper folding.

Therefore, eukaryotic expression systems have been developed of which the methylotrophic fungus Komagataella phaffii, also known as Pichia pastoris, is one example of a eukaryotic expression system developed for production of heterologous proteins and peptides in industrial settings.

Komagataella phaffii has several advantages over other expression systems. For example, it can grow on simple, inexpensive media with a high growth rate and can even grow in shake flasks or a fermenter. It can grow to extremely high cell densities and usually gives much better yields than other eukaryotic expression systems such as Chinese hamster ovary cells.

Expressing the protein or peptide of interest using Komagataella phaffii, however, has certain drawbacks. For example, not every, in particular human, protein or peptide can be successfully expressed using Komagataella phaffii, for reasons often unknown. Sometimes the yield is not satisfactory and production using Komagataella phaffii can thus be more cost intensive than the actual value of the obtained protein or peptide.

It is known to increase the yield of a protein by introducing several copies of the gene encoding for the protein of interest into Komagataella phaffii. For example, the introduction of several copies of human serum albumin into the cell was used for increasing the yield of this protein, wherein the copies were integrated into the AOX1 or HIS4 locus (WO 92/13951A1). The same technique has been used for the expression of antibodies as an example for multi-subunit proteins in Komagataella phaffii, wherein the coding nucleic acid sequences were integrated into the GAP, AOX1, AOX TT, ARG, OCH1, URA5, HIS4 TT or HIS4 locus (WO 2013/028635A1).

It is therefore a technical problem underlying the present invention to overcome the aforementioned problems and in particular to provide an increased yield for the expression of a peptide, in particular protein, of interest in a fungal cell, in particular a methylotrophic fungal cell, in particular Komagataella phaffii.

It is, thus, one technical problem underlying the claimed teaching to provide means and methods to provide an increased yield in protein expression and preferably to provide an efficient and economical way to provide a high yield expression of a peptide of interest in question, in particular in Komagataella phaffii, in particular of a collagen peptide.

This technical problem is solved by the subject-matter of the independent claims. In particular, this problem is solved by the introduction of at least one exogenous nucleic acid sequence, in particular a recombinant gene, coding for a peptide, in particular protein, of interest into the yurl gene of a fungal cell, in particular methylotrophic fungal cell, in particular Komagataella phaffii, in particular introducing several copies of the recombinant gene into the yurl gene. Thus, the present invention solves its technical problem by providing a fungal cell, which fungal cell comprises at least one copy of an exogenous nucleic acid sequence of interest stably integrated into the yurl gene of the fungal cell, which exogenous nucleic acid sequence of interest encodes a recombinant peptide of interest, in particular a collagen peptide, and by providing means, in particular expression vectors, and processes to obtain and use such a fungal cell.

Without being bound by theory, the inventors have surprisingly found that the integration of a nucleic acid sequence coding for a peptide of interest into the yurl gene of a fungal cell, in particular Komagataella phaffii, leads to a high yield expression of this peptide. Furthermore, this effect is further enhanced by the integration of more than one copy, in particular three, four, five or six copies, of the nucleic acid sequence encoding the peptide of interest. In a particularly preferred embodiment of the present invention, the high yield expression of the peptide of interest is highly stably over numerous cycles of cultivation.

Yur1 is a putative mannosyltransferase and is located in the Golgi apparatus. There it is involved in N-linked protein glycosylation, namely in the elongation of mannose chains playing a role in the glycosylation of cell wall mannoproteins. Yur1 is a member of the KTR family including enzymes with overlapPIN4Hg specificities thus it is a possibly redundant enzyme. The protein consists of a short N-terminal cytoplasmic region, a helical signal anchor ("single pass membrane II protein"), and the catalytic region located in the Golgi apparatus.

The present invention, thus, pertains to a fungal cell, which fungal cell comprises at least one copy of an exogenous nucleic acid sequence of interest stably integrated into the yurl gene of the fungal cell, which exogenous nucleic acid sequence of interest encodes a recombinant peptide of interest. The fungal cell of the present invention is particularly characterized by an increased expression of a recombinant peptide of interest due to the integration of the at least one copy of the exogenous nucleic acid into the yurl gene of said fungal cell, wherein the exogenous nucleic acid comprises a coding sequence for the peptide of interest.

The present invention, thus, provides the unexpected and advantageous teaching that a fungal cell, which comprises stably integrated into the yurl gene of said fungal cell at least one copy of an exogenous nucleic acid sequence of interest, shows a particularly high expression, in particular high expression rate and/or high expression yield of the recombinant peptide of interest being encoded by said at least one exogenous nucleic acid sequence of interest. Thus, the use of the present fungal cells in methods to produce a recombinant peptide of interest results in a highly increased yield of the recombinant peptide of interest and, thus, enables a very efficient and high yield production of a peptide of interest. Thus, the present invention provides high producer fungal cells for the production of recombinant peptides of interest, methods to produce these fungal cells as well as methods and uses of said high producer fungal cells in the production of recombinant peptides of interest.

The present invention is particularly advantageous also, because the present teaching to employ the yurl gene as a target locus for foreign gene integration and expression allows unexpectedly the integration of a high number of copies of the foreign gene into the target locus, which in turn enables a favourable multicopy expression.

Furthermore, the present fungal cells are advantageous insofar as the stably integrated exogenous nucleic acid sequence remains stably integrated in the genome of the fungal cells so as to allow an efficient long-term production with high yields.

In a preferred embodiment of the present invention, the yurl gene of the fungal cell is located on chromosome 2 of the fungal cell. Thus, the stable integration of the at least one copy of an exogenous nucleic acid sequence of interest takes place and is located in a preferred embodiment of the present invention on chromosome 2 of the fungal cell, in particular of Komagataella phaffii.

In a preferred embodiment of the present invention, the at least one copy of an exogenous nucleic acid sequence of interest is integrated in a yurl gene, which yurl gene is in a non-native position on the chromosome, in particular chromosome 2, of the fungal cell, preferably Komagataella phaffii. Such particularly preferred embodiments show a high stability of high yield expression.

In a furthermore preferred embodiment of the present invention, the non-native position of the yurl gene is a central position on the chromosome, in particular on chromosome 2 of Komagataella phaffii, located in the 3'-direction of the native position of the yurl gene on chromosome 2.

In a preferred embodiment of the present invention, the fungal cell comprises in addition to the at least one copy of the exogenous nucleic acid sequence of interest at least one copy, preferably two copies, most preferably three copies, of an exogenous nucleic acid sequence encoding a P4H (Prolyl-4-hydroxylase) or one copy, preferably two copies, of an exogenous nucleic acid sequence encoding a PIN4H (Proline-4-hydroxylase) or both of these nucleic acid sequences, which at least one copy is stably integrated into at least one chromosome of the fungal cell.

In a preferred embodiment of the present invention, the P4H can be a mammalian, preferably bovine, P4H or a viral P4H, in particular from the Mimivirus of Acanthamoeba polyphaga, that means a Mimi P4H.

In a preferred embodiment of the present invention, the fungal cell comprises in addition to the at least one copy of the exogenous nucleic acid sequence of interest at least one copy, preferably two copies, of an exogenous nucleic acid sequence encoding a lysyl hydroxylase, which at least one copy is stably integrated into at least one chromosome of the fungal cell.

In a preferred embodiment of the present invention, the fungal cell, thus, not only comprises the at least one copy of an exogenous nucleic acid sequence of interest stably integrated in the yurl gene of the fungal cell, but in addition also contains at least one copy of an exogenous nucleic acid sequence, which exogenous nucleic acid sequence is encoding a hydroxylase, in particular a P4H, a PIN4H or a lysyl hydroxylase or two or all three of them. In a preferred embodiment of the present invention, the fungal cell provided by the present teaching is, thus, characterized by the ability to hydroxylate peptides produced by said cell, in particular the peptide of interest encoded by the at least one copy of an exogenous nucleic acid sequence of interest. Surprisingly and most advantageously, it was found by the present inventors that the fungal cell, which comprises at least one copy of an exogenous nucleic acid sequence of interest stably integrated in the yurl gene of the fungal cells and further comprising at least one, in particular at least two, copies of an exogenous nucleic acid sequence encoding a P4H expresses a highly hydroxylated peptide, in particular collagen peptide.

In a preferred embodiment of the present invention, the fungal cell comprises the at least one copy of an exogenous nucleic acid sequence encoding the P4H, the PIN4H, the lysyl hydroxylase or two of these or all three on another chromosome than the at least one copy of an exogenous nucleic acid sequence of interest, in particular not on chromosome 2 of Komagataella phaffii.

In a furthermore preferred embodiment of the present invention, the at least one exogenous nucleic acid sequence encoding the P4H, the PIN4H, the lysyl hydroxylase or two of these or all three are not integrated in the yurl gene of the fungal cell.

In a particularly preferred embodiment of the present invention, the fungal cell comprises at least two copies of an exogenous nucleic acid sequence encoding P4H, two copies of an exogenous nucleic acid sequence encoding a PIN4H or one copy of an exogenous nucleic acid sequence encoding a P4H and one copy of an exogenous nucleic acid sequence encoding a PIN4H. In a preferred embodiment of this embodiment, the at least two copies can be integrated separately or sequentially together, in particular in the same orientation on the chromosome.

In a particularly preferred embodiment of the present invention, the fungal cell comprises at least two copies of an exogenous nucleic acid sequence encoding a lysyl hydroxylase or one copy of an exogenous nucleic acid sequence encoding a P4H and one copy of an exogenous nucleic acid sequence encoding a lysyl hydroxylase or one copy of an exogenous nucleic acid sequence encoding a PIN4H and one copy of an exogenous nucleic acid sequence encoding a lysyl hydroxylase. In a preferred embodiment of this embodiment, the at least two copies can be integrated separately or sequentially together, in particular in the same orientation on the chromosome.

In a particularly preferred embodiment of the present invention, the fungal cell comprises at least one copy of an exogenous nucleic acid sequence encoding a P4H, one copy of an exogenous nucleic acid sequence encoding a PIN4H and one copy of an exogenous nucleic acid sequence encoding a lysyl hydroxylase. In a preferred embodiment of this embodiment, the at least three copies can be integrated separately or sequentially together, in particular in the same orientation on the chromosome.

In a preferred embodiment of the present invention, the fungal cell comprises at least two, preferably at least three, preferably at least four, preferably at least five, most preferably five, most preferably six copies of an exogenous nucleic acid sequence of interest in the yurl gene. In a preferred embodiment, the multiple copies are preferably integrated in the same orientation on the chromosome. In a preferred embodiment at least two, in particular at least three, in particular at least four, in particular at least five, in particular at least 6, copies are integrated in the yurl gene in the same orientation on the chromosome.

In a preferred embodiment of the present invention, the fungal cell comprises preferably at least three, in particular at least five copies, preferably 3, 4, 5 or 6 copies, of an exogenous nucleic acid sequence of interest encoding the peptide of interest in the yurl gene and one or two copies of a P4H encoding nucleic acid sequence. Preferably, the one or two copies of a P4H encoding nucleic acid sequence are not integrated in the yurl gene.

In a particularly preferred embodiment of the present invention, at least one copy, preferably the two copies, of the P4H encoding nucleic acid sequence is integrated into the AOX gene locus of the fungal cell, in particular Komagataella phaffii.

In a preferred embodiment of the present invention, the at least one copy of the exogenous nucleic acid sequence of interest is stably integrated at the 3' end of the protein coding region of a yurl gene, preferably a functional yurl gene.

In a preferred embodiment of the present invention, the at least one copy of the exogenous nucleic acid sequence of interest is stably integrated in the same orientation on the chromosome as the yurl gene. Preferably, thus, the protein coding region of yurl and the coding sequence for the peptide of interest are present in the same orientation on the chromosome.

In a preferred embodiment of the present invention, the exogenous nucleic acid sequence of interest encoding a recombinant peptide of interest is encoding a recombinant peptide selected from the group consisting of collagen, α-lactalbumin, lactoferrin, albumin, elastin and the green fluorescent protein (GFP).

In a preferred embodiment of the present invention, the exogenous nucleic acid sequence of interest encoding a recombinant peptide of interest is encoding a recombinant peptide selected from the group consisting of collagen, α-lactalbumin, lactoferrin, albumin and elastin.

In a preferred embodiment of the present invention, the exogenous nucleic acid sequence of interest encoding a recombinant peptide of interest is encoding at least one collagen peptide.

In a preferred embodiment, the collagen peptide has a molecular mass from 0,1 to 300, preferably 0,5 to 300, preferably 1 to 290, preferably 10 to 200, preferably 30 to 120, preferably 40 to 110, preferably 45 to 90, preferably 90 kDa, preferably 35 to 60 kDa, in particular 45 kDa. In a furthermore preferred embodiment, the collagen peptide has a molecular mass from 0,5 to 20, preferably 1 to 18, preferably 2 to 17, preferably 2 to 15 kDa.

In a particularly preferred embodiment of the present invention, the exogenous nucleic acid sequence of interest encoding a recombinant peptide of interest is encoding a collagen peptide, comprising, in particular consisting of, the nucleic acid sequence of SEQ ID No. 1.

In a preferred embodiment of the present invention, the exogenous nucleic acid sequence of interest comprises the sequence of SEQ ID No. 1 or of a functionally equivalent sequence variant thereof, the variant having at least 80% sequence identity to SEQ ID No. 1 or the variant being capable of hybridizing under low, medium or high stringency conditions with the nucleic acid sequence of SEQ ID No. 1 or its complementary strand or consists of any of them.

In a preferred embodiment of the present invention, the exogenous nucleic acid sequence of interest is the sequence of SEQ ID No. 1 or of a functionally equivalent sequence variant thereof, the variant having at least 80% sequence identity to SEQ ID No. 1 or the variant being capable of hybridizing under low, medium or high stringency conditions with the nucleic acid sequence of SEQ ID No. 1 or its complementary strand.

In a preferred embodiment of the present invention, the exogenous nucleic acid sequence of interest is contained in the sequence of SEQ ID No. 10, SEQ ID No. 12, SEQ ID No. 17 or is a functionally equivalent sequence variant thereof, the variant having at least 80% sequence identity to SEQ ID No. 10, 12 or 17, or the variant being capable of hybridizing under low, medium or high stringency conditions with the nucleic acid sequence of SEQ ID No. 10, 12 or 17 or its complementary strand, in particular is contained in the sequence of SEQ ID No. 12 or 17 or is a functionally equivalent sequence variant thereof, the variant having at least 80% sequence identity to SEQ ID No. 12 or 17 or the variant being capable of hybridizing under low, medium or high stringency conditions with the nucleic acid sequence of SEQ ID No. 12, 17 or its complementary strand.

The present invention in a preferred embodiment relates to a fungal cell, wherein the exogenous nucleic acid sequence of interest encoding a recombinant peptide of interest is encoding a collagen peptide, in particular a type I collagen peptide, in particular an alpha-1-type-I collagen, in particular having the nucleic acid sequence of SEQ ID No. 1 or of a functionally equivalent sequence variant thereof, the variant having at least 80% sequence identity to SEQ ID No. 1 or the variant being capable of hybridizing under low, medium or high stringency conditions with the nucleic acid sequence of SEQ ID No. 1 or its complementary strand. Preferably, the collagen is a bovine collagen peptide, in particular a bovine type I collagen peptide, preferably an alpha-1-type-I collagen.

In a particularly preferred embodiment of the present invention, the exogenous nucleic acid sequence of interest encoding a recombinant peptide of interest is encoding a collagen peptide of the Col1A1 of Bos taurus, in particular a fragment of Col1A1, in particular a fragment with a molecular mass of 45 kDa.

In a particularly preferred embodiment of the present invention, the exogenous nucleic acid sequence of interest encoding a recombinant peptide of interest is encoding a collagen peptide of the amino acid sequence SEQ ID No. 2 or a fragment thereof.

In a particularly preferred embodiment of the present invention, the exogenous nucleic acid sequence of interest encoding a recombinant peptide of interest is encoding a peptide, in particular collagen peptide, wherein the codon usage for encoding the peptide, in particular collagen peptide, has been optimized to ensure an optimal expression in the fungal cell in which the nucleic acid sequence is to be integrated. For example, for increasing the expression, codons are used which are more frequently used in the specific species of fungal cell, but which code for the same amino acid. Thus, the optimization of the codon usage can change the nucleic acid sequence, but the amino acid sequence for which the nucleic acid is coding will remain unchanged.

The present invention, thus, relates in particular to an exogenous nucleic acid sequence of interest encoding a recombinant peptide of interest, which exogenous nucleic acid sequence of interest is codon-optimized for the expression in a particular host, in particular fungal host cell, in particular Komagataella phaffii.

The present invention, thus, relates in particular to an exogenous nucleic acid sequence of interest encoding a recombinant collagen peptide, which exogenous nucleic acid sequence of interest is codon-optimized for the expression in a particular host, in particular fungal host cell, in particular Komagataella phaffii. Preferably, the exogenous nucleic acid sequence of interest is a codon-optimized bovine collal gene.

In a preferred embodiment of the present invention the fungal cell is selected from the group consisting of a Candida cell, Hansenula cell, Torulopsis cell, Saccharomyces cell, Kluyveromyces cell, Cyberlindnera cell, Rhodotorula cell, Yarrowia cell, Lipomyces cell and Komagataella cell. In a particularly preferred embodiment of the present invention the fungal cell is a methylotrophic fungal cell. In a particularly preferred embodiment of the present invention, the fungal cell is a Komagataella phaffii cell.

In a particularly preferred embodiment of the present invention, the fungal cell, in particular Komagataella phaffii cell, comprises at least one copy, preferably at least three, in particular at least four, in particular at least five copies, preferably 3, 4, 5 or 6 copies, of an exogenous nucleic acid sequence of interest stably integrated in the yurl gene of the fungal cell and wherein the exogenous nucleic acid sequence of interest is encoding a collagen peptide.

In a particularly preferred embodiment of the present invention, the fungal cell, in particular Komagataella phaffii cell, comprises at least three copies, preferably three copies, of an exogenous nucleic acid sequence of interest stably integrated in the yurl gene of the fungal cell and wherein the exogenous nucleic acid sequence of interest is encoding a collagen peptide.

In a particularly preferred embodiment of the present invention, the fungal cell, in particular Komagataella phaffii cell, comprises at least four copies, preferably four copies, of an exogenous nucleic acid sequence of interest stably integrated in the yurl gene of the fungal cell and wherein the exogenous nucleic acid sequence of interest is encoding a collagen peptide.

In a particularly preferred embodiment of the present invention, the fungal cell, in particular Komagataella phaffii cell, comprises at least five copies, preferably five copies, of an exogenous nucleic acid sequence of interest stably integrated in the yurl gene of the fungal cell and wherein the exogenous nucleic acid sequence of interest is encoding a collagen peptide.

In a particularly preferred embodiment of the present invention, the fungal cell, in particular Komagataella phaffii cell, comprises at least six copies, preferably six copies, of an exogenous nucleic acid sequence of interest stably integrated in the yurl gene of the fungal cell and wherein the exogenous nucleic acid sequence of interest is encoding a collagen peptide.

In a particularly preferred embodiment of the present invention, the fungal cell, in particular Komagataella phaffii cell, comprises at least one copy, preferably at least three, preferably at least four, preferably at least five, preferably at least six copies of an exogenous nucleic acid sequence of interest stably integrated in the yurl gene of the fungal cell and at least one, preferably two, copies of an exogenous nucleic acid sequence encoding a P4H, a PIN4H or a lysyl hydroxylase, or two or three of them, which at least one copy of the exogenous nucleic acid sequence encoding a P4H, PIN4H, lysyl hydroxylase or two or three of them is or are stably integrated into at least one chromosome of the fungal cell, and wherein the exogenous nucleic acid sequence of interest is encoding a collagen peptide.

Most advantageously, in a preferred embodiment a fungal cell of the present invention is able to produce a hydroxylated collagen peptide.

In a particularly preferred embodiment, the hydroxylation degree of the hydroxylated peptide, in particular collagen peptide, is in particular from 20 to 100, in particular from 30 to 100, in particular from 40 to 100, in particular from 50 to 100, in particular from 60 to 100, in particular from 70 to 100, in particular from 80 to 100, preferably 90 to 100, in particular 95 to 100, preferably 100 %.

In a particularly preferred embodiment, the hydroxylation degree of the hydroxylated peptide, in particular collagen peptide, with respect to hydroxy proline is in particular from 20 to 100, in particular from 30 to 100, in particular from 40 to 100, in particular from 50 to 100, in particular from 60 to 100, in particular from 70 to 100, in particular from 80 to 100, in particular from 85 to 100, preferably 90 to 100, in particular 95 to 100, preferably 100 %.

The hydroxylation degree is the proportion of hydroxylated proline residues in a peptide of the present invention in relation to the number of hydroxylated proline residues in a naturally occurring 45 kDa (516 amino acids, with 51 hydroxyprolines (42%) and 71 prolines (58%) collagen peptide, in particular Protein UniProtKB - P02457 CO1A1_CHICK (SEQ ID No. 22).

The hydroxylation degree is calculated as c_{Hyp}^{∗}100/[(c_{Hyp}+c_{Proline})^{∗}42]. Accordingly, a hydroxylation degree of 100 % corresponds to a full native-like hydroxylation, that means 42 % hydroxylated prolines on the basis of all prolines present in the peptide.

In a particularly preferred embodiment of the present invention, the fungal cell, in particular Komagataella phaffii cell, comprises at least three copies, preferably three or four copies, of an exogenous nucleic acid sequence of interest stably integrated in the yurl gene of the fungal cell and at least one, preferably one or two copies, of an exogenous nucleic acid sequence encoding a P4H, which at least two copies of the exogenous nucleic acid sequence encoding a P4H are stably integrated into at least one chromosome of the fungal cell, and wherein the exogenous nucleic acid sequence of interest is encoding a collagen peptide. Most advantageously, such a fungal cell is able to produce a hydroxylated collagen peptide.

In a particularly preferred embodiment of the present invention, the fungal cell, in particular Komagataella phaffii cell, comprises at least five copies, preferably five or six copies, of an exogenous nucleic acid sequence of interest stably integrated in the yurl gene of the fungal cell and at least one, preferably one or two copies, of an exogenous nucleic acid sequence encoding a P4H, which at least two copies of the exogenous nucleic acid sequence encoding a P4H are stably integrated into at least one chromosome of the fungal cell, and wherein the exogenous nucleic acid sequence of interest is encoding a collagen peptide. Most advantageously, such a fungal cell is able to produce a hydroxylated collagen peptide.

In a particularly preferred embodiment, the peptide of interest, in particular the collagen peptide comprises hydroxylated prolines, that means hydroxy prolines.

In a particularly preferred embodiment, the peptide of interest, in particular the collagen peptide comprises hydroxylated lysines, that means hydroxy lysines.

In a particularly preferred embodiment, the peptide of interest, in particular the collagen peptide comprises hydroxylated prolines and lysines, that means hydroxy prolines and hydroxy lysines.

In a particularly preferred embodiment, the hydroxylation degree of the hydroxylated peptide, in particular, the collagen peptide, is from 70 to 100, preferably from 75 to 100, in particular from 80 to 100, preferably from 85 to 100, preferably from 90 to 100, preferably from 95 to 100, preferably from 85 to 99, preferably from 90 to 99 %.

In a particularly preferred embodiment, the hydroxylation degree of the hydroxylated peptide, in particular, the collagen peptide, with respect to hydroxy proline is in particular from 20 to 100, in particular from 30 to 100, in particular from 40 to 100, in particular from 50 to 100, in particular from 60 to 100, in particular from 70 to 100, preferably from 75 to 100, in particular from 80 to 100, preferably from 85 to 100, preferably from 90 to 100, preferably from 95 to 100, preferably from 85 to 99, preferably from 90 to 99 %.

In a preferred embodiment of the present invention, the at least one copy of an exogenous nucleic acid sequence of interest is integrated in a yurl gene, which yurl gene is in a non-native position on the chromosome, in particular chromosome 2, of the fungal cell, preferably Komagataella phaffii.

In a preferred embodiment, a fungal cell, wherein the at least one copy of an exogenous nucleic acid sequence of interest is integrated in a yurl gene, which yurl gene is in a non-native position on the chromosome, in particular chromosome 2, of the fungal cell, preferably Komagataella phaffii, is Komagataella phaffii 451-1 (DSM 33955, deposited on 28.07.2021), Komagataella phaffii 451-2 (DSM 33956, deposited on 28.07.2021), Komagataella phaffii 451-3 (DSM 33957, deposited on 28.07.2021) or a further derivative of Komagataella phaffii 45I-1.

In a furthermore preferred embodiment of the present invention, the non-native position of the yurl gene is a central position on the chromosome, preferably located on chromosome 2 in the 3'-direction of the native position of the yurl gene on chromosome 2 of Komagataella phaffii.

In a preferred embodiment, a fungal cell, wherein the at least one copy of an exogenous nucleic acid sequence of interest is integrated in a yurl gene, which yurl gene is in a central position on the chromosome, preferably located on chromosome 2 in the 3'-direction of the native position of the yurl gene on chromosome 2 of Komagataella phaffii, is Komagataella phaffii 451-1 (DSM 33955, deposited on 28.07.2021), Komagataella phaffii 451-2 (DSM 33956, deposited on 28.07.2021), Komagataella phaffii 451-3 (DSM 33957, deposited on 28.07.2021) or a further derivative of Komagataella phaffii 451-1.

In a furthermore preferred embodiment of the present invention, the fungal cell is a Komagataella phaffii cell, preferably as deposited with the DSMZ under accession numbers: Komagataella phaffii 451-1 (DSM 33955, deposited on 28.07.2021), Komagataella phaffii 451-2 (DSM 33956, deposited on 28.07.2021), Komagataella phaffii 451-3 (DSM 33957, deposited on 28.07.2021), a further derivate of Komagataella phaffii 451-1, Komagataella phaffii 451-4 (DSM 33958, deposited on 28.07.2021) or a derivate of Komagataella phaffii 451-4.

In a furthermore preferred embodiment of the present invention, the fungal cell is a Komagataella phaffii cell, preferably as deposited with the DSMZ under accession numbers: Komagataella phaffii 451-4 (DSM 33958, deposited on 28.07.2021) or a derivative of Komagataella phaffii 451-4.

In the context of the present invention, a "derivative of Komagataella phaffii K45I-1" is a Komagataella phaffii cell, which comprises the genome of Komagataella phaffii K45I-1, in which genome
a) at least one further exogeneous nucleic acid sequence, in particular encoding a peptide of interest or a hydroxylase and/or a further exogeneous regulatory unit is added in addition to those already being present in Komagataella phaffii 451-1, in particular Komagataella phaffii 451-2 or Komagataella phaffii 451-3, or
b) at least one of the exogeneous nucleic acid sequences, in particular encoding a peptide of interest or a hydroxylase and/or exogeneous regulatory units being present in Komagataella phaffii K45I-1 is partially or totally deleted, or
c) at least one of the exogeneous nucleic acid sequences, in particular encoding a peptide of interest or a hydroxylase and/or exogeneous regulatory units being present in Komagataella phaffii K45I-1 is partially or totally deleted and replaced by at least one other exogeneous nucleic acid sequence and/ or regulatory unit, or
d) the yurl gene is in a non-native position on chromosome 2, in particular in 3'-direction from the native position of the yurl gene on chromosome 2, preferably in a central position, preferably between the catalase locus and MRPL4, preferably at positions 255125 to 1577812, most preferably in an inverse orientation with respect to the native orientation of the yurl in its native position, or
e) the yurl gene is totally or partially deleted, in particular is truncated at its 3'end and in which truncated yurl gene, preferably in its non-native position on chromosome 2, solely the nucleotide sequence of SEQ ID No. 15 is present and the native nucleotides at positions 1168 to 1191 of SEQ ID No. 13 are thus deleted, or
f) a combination of any of a) to e) occurred.

In the context of the present invention, a "derivative of Komagataella phaffii K45I-4" is a Komagataella phaffii cell, which comprises the genome of Komagataella phaffii K45I-4, in which genome
a) at least one further exogeneous nucleic acid sequence, in particular encoding a peptide of interest or a hydroxylase and/or a further exogeneous regulatory unit is added in addition to those already being present in Komagataella phaffii K45I-4, or
b) at least one of the exogeneous nucleic acid sequences, in particular encoding a peptide of interest and/or exogeneous regulatory units being present in Komagataella phaffii K45I-4 is partially or totally deleted, or
c) at least one of the exogeneous nucleic acid sequences, in particular encoding a peptide of interest and/or exogeneous regulatory units being present in Komagataella phaffii K45I-4 is partially or totally deleted and replaced by at least one other exogeneous nucleic acid sequence and/ or regulatory unit, or
d) the yurl gene is totally or partially deleted, or
e) a combination of any of a) to d) occurred.

In a preferred embodiment, the invention relates to a fungal cell of Komagataella phaffii as deposited with the Leibniz-Institut (DSMZ-Deutsche Sammlung von Mikroorganismen und Zellkulturen GmbH, Germany) in accordance with the provisions of the Budapest Treaty:
Komagataella phaffii 451-1 = DSM 33955, deposited on 28.07.2021.

In a preferred embodiment, the invention also relates to a fungal cell of Komagataella phaffii as deposited with the DSMZ in accordance with the provisions of the Budapest Treaty:
Komagataella phaffii 451-2 = DSM 33956, deposited on 28.07.2021.

In a preferred embodiment, the invention also relates to a fungal cell of Komagataella phaffii as deposited with the DSMZ in accordance with the provisions of the Budapest Treaty:
Komagataella phaffii 451-3 = DSM 33957, deposited on 28.07.2021.

In a preferred embodiment, the invention also relates to a fungal cell of Komagataella phaffii as deposited with the DSZM in accordance with the provisions of the Budapest Treaty:
Komagataella phaffii 451-4 = DSM 33958, deposited on 28.07.2021.

In a preferred embodiment of the present invention, the recombinant peptide of interest encoded by the exogenous nucleic acid sequence of interest is a fusion protein, in particular comprising a collagen peptide and, in N-terminal direction, in C-terminal direction or in both directions of the amino acid sequence of the collagen peptide, at least one, in particular at least two, further functional peptides, in particular complete proteins or protein domains.

In a preferred embodiment of the present invention, the functional peptide is a secretion signal, in particular from a mating factor alpha (α), in particular from Saccharomyces cerevisiae. Preferably, the secretion signal from a mating factor comprises a pre- and a pro-sequence.

Thus, the present invention preferably relates to a fungal cell, wherein the recombinant peptide of interest encoded by the exogenous nucleic acid sequence of interest is a fusion protein, in particular comprising a collagen peptide, which is in N- and/or C-terminal direction fused to one or more further functional peptides.

Thus, a fusion protein in the context of the present invention comprises at least two elements, that means one element, which is a first peptide and a second element, which is a second peptide and which are fused due to their expression from an exogenous nucleic acid sequence encoding in one open reading frame the at least two elements, that means peptides.

In a preferred embodiment of the present invention, the recombinant peptide of interest encoded by the exogenous nucleic acid sequence of interest is a fusion protein comprising as a first element, as seen from the N-terminus, a functional peptide, in particular a secretion signal, in particular from a mating factor (MF) α, preferably a mating factor α from Saccharomyces cerevisiae, and as a second element a collagen peptide and, optionally, as a third element a further functional peptide, in particular proteins or protein domains.

In a preferred embodiment, the secretion signal from MF is the MF alpha nucleotide sequence such as identified in SEQ ID No. 20 (1-57 pre-sequence, 58-267 pro-sequence).

In a preferred embodiment, the secretion signal from MF is the MF alpha amino acid sequence such as identified in SEQ ID No. 21 (MF alpha (1-19 pre-sequence, 20-89 pro-sequence).

In a preferred embodiment of the present invention, the exogenous nucleic acid sequence of interest comprises, in addition to the coding region for the recombinant peptide of interest, at least one, preferably at least two, preferably at least three, preferably two, preferably three, regulatory units, in particular a promoter, an enhancer, a silencer and/or a terminator.

In a preferred embodiment, the coding region for the recombinant peptide of interest is functionally linked to said at least one, preferably two, preferably three regulatory units.

In a preferred embodiment of the present invention, the nucleic acid sequence encoding a P4H, a PIN4H or a lysyl hydroxylase comprises in addition to the coding sequence for the P4H, PIN4H or lysyl hydroxylase at least one, preferably at least two, preferably at least three, preferably two, preferably three, regulatory units, in particular a promoter, an enhancer, a silencer and/or a terminator.

In a preferred embodiment, the coding region for the P4H, PIN4H or lysyl hydroxylase is functionally linked to said at least one, preferably two, preferably three regulatory units.

In a particularly preferred embodiment of the present invention, the promoter is an inducible promoter, in particular a methanol-inducible promoter.

In a particularly preferred embodiment of the present invention, the promoter is a constitutive promoter.

In a particularly preferred embodiment of the present invention, the promoter is a derepressed promoter.

In a particularly preferred embodiment of the present invention, the promoter is a promoter selected from the group consisting of pCAT, pAOX, pDAS1, pDAS2, pFDH1, pGAP and pDF.

These promoters are described for instance in Vogl et al., 2016, DOI: 10.1021/acssynbio.5b00199 and Vogl et al., 2018, DOI:10.1038/s41467-018-05915-w and Fischer et al., 2019, DOI:10.3791/58589.

In a particularly preferred embodiment of the present invention, the promoter is a promoter from a catalase gene, in particular from a catalase of Komagataella phaffii, in particular pCAT (also termed pDC, pCAT1 or pCAT1-500).

This promoter is naturally located 500 bp upstream of the catalase gene and is described for instance in Vogl et al., 2018, DOI:10.1038/s41467-018-05915-w and Fischer et al., 2019, DOI:10.3791/58589.

In a particularly preferred embodiment of the present invention, the promoter is a promoter from a glyceraldehyde 3-phosphate (GAP) gene, in particular the GAP promoter of Komagataella phaffii.

In a particularly preferred embodiment of the present invention, the promoter is a PDC promoter from Komagataella phaffii.

In a particularly preferred embodiment of the present invention, the terminator is a terminator from the AOX (alcohol oxidase) gene, in particular AOX of Komagataella phaffii.

In a preferred embodiment of the present invention, the exogenous nucleic acid sequence of interest further comprises a nucleic acid sequence encoding a selectable marker, in particular a positive selectable marker, in particular an antibiotic resistance, preferably zeocin resistance, hygromycin resistance, geniticin resistance or a negative selectable marker. In a preferred embodiment, the selectable marker comprises a promoter, in particular an ILV5 and/or EM72 promoter, a protein coding sequence for a protein for conveying the selectable advantage or disadvantage, in particular a protein binding and thereby neutralizing zeocin, and a terminator.

In a preferred embodiment of the present invention, the exogenous nucleic acid sequence of interest further comprises a nucleic acid sequence encoding for an origin of replication (ori), in particular a prokaryotic origin of replication, in particular a pUC origin of replication.

In a preferred embodiment of the present invention, the exogenous nucleic acid sequence of interest has been integrated into the yurl gene by homologous recombination, in particular employing Zinc-finger-nucleases, TALEN, Cre/loxP system or CRISPR/CAS for introduction of a double strand break.

In a preferred embodiment of the present invention, the exogenous nucleic acid sequence of interest has been integrated into the yurl gene by a non-homologous recombination, in particular employing Zinc-finger-nucleases, TALEN, Cre/loxP system or CRISPR/CAS for introduction of a double strand break.

In a preferred embodiment of the present invention, the exogenous nucleic acid sequence encoding a P4H, a PIN4H or a lysyl hydroxylase has been integrated into the genome by homologous recombination, in particular employing Zinc-finger-nucleases, TALEN, Cre/loxP system or CRISPR/CAS for introduction of a double strand break.

In a preferred embodiment of the present invention, the exogenous nucleic acid sequence encoding a P4H, a PIN4H or a lysyl hydroxylase has been integrated into the genome by a non-homologous recombination, in particular employing Zinc-finger-nucleases, TALEN, Cre/loxP system or CRISPR/CAS for introduction of a double strand break.

In a furthermore preferred embodiment, the present invention provides a process for producing a fungal cell according to the present invention, in particular a fungal cell comprising at least one copy of an exogenous nucleic acid sequence of interest stably integrated into the genomic locus of the yurl gene of the fungal cell, which exogenous nucleic acid sequence of interest encodes a recombinant peptide of interest, which process comprises
x) providing a fungal host cell and at least one expression vector comprising an at least one expression cassette, which expression cassette comprises at least one, preferably one, exogenous nucleic acid sequence of interest, in particular encoding a collagen peptide,
y) transforming the fungal host cell with the at least one expression vector under appropriate conditions so as to effect integration of the at least one copy of an exogenous nucleic acid sequence stably into the yurl gene, and
z) obtaining the fungal cell.

In a preferred embodiment of the present invention, there is, thus, provided a process to produce a fungal cell of the present invention.

In a preferred embodiment of the present invention, said process employs a fungal host cell selected from the group consisting of a Candida cell, Hansenula cell, Torulopsis cell, Kluyveromyces cell, Cyberlindnera cell, Rhodotorula cell, Yarrowia cell, Lipomyces cell, Saccharomyces cell and Komagataella cell. In a particularly preferred embodiment of the present invention the fungal host cell is a methylotrophic fungal cell. In a particularly preferred embodiment of the present invention, the fungal host cell is a Komagataella phaffii cell. In a particularly preferred embodiment of the present invention, the fungal host cell is Komagataella phaffii strain CBS7435, available from the ATCC under the number ATCC76273.

Furthermore, in a preferred embodiment, said process uses an expression vector, which expression vector is preferably a plasmid or virus.

In a furthermore preferred embodiment of the present invention, the expression vector comprises one expression cassette, which expression cassette preferably comprises at least one copy, preferably one copy, of an exogenous nucleic acid sequence of interest.

In a preferred embodiment of the present invention, at least one, preferably more than one expression vector, expression cassette and/or a fragment thereof, in particular at least three, preferably at least at least four, preferably at least five, preferably at least six, copies of an expression vector, expression cassette and/or a fragment thereof, each of them comprising at least one copy, preferably one copy, of an exogenous nucleic acid sequence of interest are integrated stably into the yurl gene.

In a preferred embodiment of the present invention, said expression vector also comprises an expression cassette, which expression cassette comprises at least one copy of an exogenous nucleic acid sequence encoding a P4H, a PIN4H or both.

In a preferred embodiment of the present invention, said expression vector also comprises an expression cassette, which expression cassette comprises at least one copy of an exogenous nucleic acid sequence encoding a lysyl hydroxylase.

In a preferred embodiment of the present invention in step x), at least two different expression vectors are provided, in particular one expression vector comprising at least one, preferably one expression cassette, which expression cassette comprises at least one exogenous nucleic acid sequence of interest, and a further expression vector comprising at least one, preferably one, expression cassette comprising at least one copy of a nucleic acid sequence encoding a P4H or PIN4H and optionally a further expression vector comprising an expression cassette, which expression cassette comprises at least one exogenous nucleic acid sequence encoding a lysyl hydroxylase.

In a preferred embodiment of the present invention, the at least one copy of an exogenous nucleic acid sequence of interest is functionally linked in the expression cassette to at least one, preferably at least two, preferably at least three, preferably two, preferably three regulatory units, in particular a promoter, an enhancer, a silencer and/or a terminator. In a preferred embodiment, the exogenous nucleic acid sequence of interest encodes a fusion protein comprising a collagen peptide and a secretion signal, in particular from mating factor α from Saccharomyces cerevisiae.

In a preferred embodiment of the present invention, the at least one exogenous nucleic acid sequence encoding a P4H or a PIN4H is functionally linked in the expression cassette to at least one, preferably at least two, preferably at least three, preferably two, preferably three, regulatory units, in particular a promoter, an enhancer, a silencer and/or a terminator.

In a preferred embodiment of the present invention, the at least one exogenous nucleic acid sequence encoding a lysyl hydroxylase is functionally linked in the expression cassette to at least one regulatory unit, preferably at least two, preferably at least three, preferably two, preferably three, regulatory units, in particular a promoter, an enhancer, a silencer and/or a terminator.

In a preferred embodiment of the present invention, the expression vector as provided in step x) is configured to be integrated either by non-homologous or homologous recombination, in particular of the at least one exogenous nucleic acid sequence of interest into the yurl gene.

In a preferred embodiment of the present invention, the expression vector as provided in step x) are configured to be integrated either by homologous recombination, in particular of the at least one exogenous nucleic acid sequence of interest into the yurl gene.

In a preferred embodiment of the present invention, the expression vector as provided in step x) comprises at least one nucleic acid sequence of at least 10, preferably at least 25, preferably at least 50, preferably at least 100, preferably at least 150, preferably all, nucleotides of the yurl gene, preferably as identified in SEQ ID No. 13.

Thus, the present invention also relates to an expression vector comprising an expression cassette, which expression cassette comprises at least one nucleic acid sequence of interest, in particular encoding a collagen peptide, and at least nucleic acid sequence of at least 10, preferably at least 25, preferably at least 50, preferably at least 100, preferably at least 150, preferably all, nucleotides of the yurl gene, preferably as identified in SEQ ID No. 13.

The present invention also relates to a host cell containing an expression vector of the present invention, in particular an expression vector comprising an expression cassette, which expression cassette comprises at least one nucleic acid sequence of interest, in particular encoding a collagen peptide, and at least nucleic acid sequence of at least 10, preferably at least 25, preferably at least 50, preferably at least 100, preferably at least 150, preferably all, nucleotides of the yurl gene, preferably as identified in SEQ ID No. 13.

The host cell can be preferably a bacterial, fungal, plant, insect or animal cell.

In a preferred embodiment, the transformation in step y) is conducted using viral transformation methods, chemical methods, or physical methods like electroporation, biolistics or sonoporation that uses cavitation of gas bubbles produced by ultrasound to penetrate the cell membrane.

In a preferred embodiment of the present invention, the integration as effected in step y) is an integration effected by homologous recombination or by non-homologous recombination.

In a preferred embodiment of the present invention, the integration effected in step y) is effected by homologous recombination.

In a preferred embodiment of the present invention, the integration effected in step y) is effected by non-homologous recombination.

The present invention also relates to a process for producing a recombinant peptide of interest in a fungal cell, which process comprises the steps of
a) providing a fungal cell according to the invention and optionally a culture medium,
b) culturing the fungal cell under conditions suitable for the expression of the recombinant peptide of interest, and
c) obtaining the recombinant peptide of interest.

In a preferred embodiment of the present invention, the present process for producing a

recombinant peptide of interest is a process for producing a hydroxylated recombinant peptide of interest in a fungal cell, which process comprises the steps of
ax) providing a fungal cell according to the invention, which comprises at least one copy of an exogenous nucleic acid sequence of interest stably integrated into the yurl gene and at least one copy of an exogenous nucleic acid sequence encoding a P4H, PIN4H or lysyl hydroxylase, in particular a P4H, and, optionally, a culture medium,
bx) culturing the fungal cell under conditions suitable for the expression and hydroxylation of the recombinant peptide of interest, and
cx) obtaining the hydroxylated recombinant peptide of interest.

In a preferred embodiment of the present invention, the recombinant peptide of interest is a hydroxylated recombinant peptide of interest, in particular a hydroxylated collagen peptide.

In a particularly preferred embodiment, the peptide of interest, in particular the collagen peptide, comprises hydroxylated prolines, that means hydroxy prolines.

In a particularly preferred embodiment, the peptide of interest, in particular the collagen peptide, comprises hydroxylated lysines, that means hydroxy lysines.

In a particularly preferred embodiment, the peptide of interest, in particular the collagen peptide comprises hydroxylated prolines and lysines, that means hydroxy prolines and hydroxy lysines. In a preferred embodiment of the present invention, in step a) or ax) there is also provided a culture medium for cultivating the fungal cell. In a preferred embodiment of the present invention, the culture medium is Syn6, preferably such as specified in Example 9.

In a preferred embodiment of the present invention, the culture medium comprises potassium dihydrogenphosphate, diammonium sulfate, 2-(N-morpholino) ethanesulfonic acid (MES), magnesium sulfate, potassium chloride, sodium chloride, water, calcium chloride, EDTA, Ammonium iron(II) sulfate, copper(II) sulfate, zinc(II) sulfate, manganese(II) sulfate, d-Biotin, 2-propanol, thiamine chloride, nickel(II) sulfate, cobalt(II) sulfate, boric acid, potassium iodide, sodium molybdate, glucose and/or trisodium citrate.

In a particularly preferred embodiment of the present invention, the culture medium is a YPD (yeast extract peptone dextrose) medium.

In a particularly preferred embodiment of the present invention, the culture medium is a Syn6 medium, optionally comprising further additives, in particular a Syn6 medium as disclosed in Example 9.

In a preferred embodiment of the present invention, the culture medium has a pH from 4.0 to 8.0, preferably 5.0 to 7.0, preferably 6.0 to 6.5, preferably 6.0.

In a preferred embodiment of the present invention, step b) or bx) requires a culturing of the fungal cells under conditions, which are suitable for the expression of the recombinant peptide of interest and, furthermore in a preferred embodiment, suitable for the hydroxylation of the recombinant peptide of interest.

In a preferred embodiment of the present invention, the culturing of step b) or bx) is performed in a batch, semi-batch, in particular fed-batch, and/or continuous mode.

In a preferred embodiment of the present invention, the hydroxylation may occur during or subsequent to the expression of the recombinant peptide of interest. Preferably, the use of a P4H results in a post-translational hydroxylation, whereas the use of a PIN4H relates to a hydroxylation during translation.

In a preferred embodiment of the present invention, step c) or cx) is a step of isolating the recombinant peptide of interest from the fungal cell.

In a preferred embodiment of the present invention, step c) or cx) is a step of isolating the recombinant peptide of interest from the culture medium.

In a preferred embodiment of the present invention, step c) or cx) is a step of isolating the recombinant peptide of interest from the fungal cell and the culture medium.

In a particularly preferred embodiment of the present invention, the fungal cell is disrupted for isolating the recombinant peptide of interest. Preferably, the disruption of the fungal cells is a mechanical disruption.

Thus, in a preferred embodiment of the present invention, the recombinant peptide of interest is obtained and provided by the present process in form of an isolated peptide of interest.

In a preferred embodiment of the present invention, the recombinant protein produced by a process of the invention is obtained by disruption of the fungal cell and subsequent purification of the peptide of interest from the disrupted cells, preferably mechanically disrupted, preferably by known protein purification means, such as centrifugation, in particular differential centrifugation and/or density gradient centrifugation, chromatography, in particular ion exchange, size exclusion or affinity chromatography, electrophoreses and/or filtration.

In a preferred embodiment the recombinant protein produced by a process of the invention is obtained from the supernatant of the culture medium, wherein the fungal cell is preferably cultivated, preferably by known protein purification means, such as centrifugation, in particular differential centrifugation and/or density gradient centrifugation, chromatography, in particular ion exchange, size exclusion or affinity chromatography, electrophoreses and/or filtration.

The present invention also relates to a recombinant peptide of interest obtainable by a process according to the invention, a cell culture comprising a fungal cell according to the invention and culture medium, and a bioreactor comprising a cell culture according to the invention.

In a preferred embodiment of the present invention, the peptide of interest obtainable, in particular obtained, by a process according to the invention is immobilized on a carrier.

In a preferred embodiment of the present invention, the recombinant peptide of interest is a collagen peptide. In this embodiment the collagen peptide preferably comprises at least one hydroxylated proline residue or at least one hydroxylated lysine residue or both at least one hydroxylated lysine residue and at least one hydroxylated proline residue. In this preferred embodiment the collagen peptide thus comprises at least one hydroxy proline or hydroxy lysine or both. In a particularly preferred embodiment of the present invention each proline and/or each lysine in a collagen peptide as recombinant peptide of interest is hydroxylated.

In a particularly preferred embodiment, the hydroxylation degree of the hydroxylated collagen peptide is in particular from 20 to 100, in particular from 30 to 100, in particular from 40 to 100, in particular from 50 to 100, in particular from 60 to 100, in particular from 70 to 100, in particular from 80 to 100, in particular from 85 to 100, preferably 90 to 100, in particular 95 to 100, preferably 100 %.

In a particularly preferred embodiment, the hydroxylation degree of the hydroxylated collagen peptide with respect to hydroxy proline is in particular from 20 to 100, in particular from 30 to 100, in particular from 40 to 100, in particular from 50 to 100, in particular from 60 to 100, in particular from 70 to 100, in particular from 80 to 100, in particular from 85 to 100, preferably 90 to 100, in particular 95 to 100, preferably 100 %.

In a preferred embodiment of the present invention, the recombinant peptide of interest is a collagen peptide for the use in a method of treating and/or preventing arthritis, in particular osteoarthritis or rheumatoid arthritis.

In a preferred embodiment of the present invention, the recombinant peptide of interest is a collagen peptide for the use in a method of enhancing muscle force.

In a preferred embodiment of the present invention, the recombinant peptide of interest is a collagen peptide for the use in a method of treating and/or preventing of a pathological condition characterized by a reduced mitochondrial activity, in particular for treating and/or preventing of a pathological condition characterized by a reduced endurance.

In a preferred embodiment of the present invention, the recombinant peptide of interest is a collagen peptide for the use in a method of stimulating fat reduction.

In a preferred embodiment of the present invention, the recombinant peptide of interest is a collagen peptide for the use in a method of body weight reduction.

In a preferred embodiment of the present invention, the recombinant peptide of interest is a collagen peptide for the use in a method of treating and/or preventing of degenerative joint diseases, in particular arthritis, rheumatoid arthritis, rheumatoid diseases, spondylitis and/or fibromyalgia.

In a preferred embodiment of the present invention, the recombinant peptide of interest is a collagen peptide for the use in a method of treating and/or preventing of diseases of the tendons or ligaments.

In a preferred embodiment of the present invention, the recombinant peptide of interest is a collagen peptide for the use in a method of treating and/or preventing of skin diseases, in particular Psoriasis vulgaris, acne, atopical dermatitis, chronic pruritus and/or rosacea.

In a preferred embodiment of the present invention, the recombinant peptide of interest is a collagen peptide for the use in a method of treating of wounds, in particular chronic wounds, acute wounds and/or burns.

In a preferred embodiment of the present invention, the recombinant peptide of interest is a collagen peptide for the use in a method of treating and/or preventing of degenerative nerve diseases.

In a preferred embodiment of the present invention, the recombinant peptide of interest is a collagen peptide for the use in a method of treating and/or preventing of dementia.

In a preferred embodiment of the present invention, the recombinant peptide of interest is a collagen peptide for the use in a method of treating and/or preventing of Alzheimer's disease.

In a preferred embodiment of the present invention, the recombinant peptide of interest is a collagen peptide for the use in a method of treating and/or preventing of a pathological condition characterized by a reduced mental performance.

In a preferred embodiment of the present invention, the recombinant peptide of interest is a collagen peptide for the use in a method of treating and/or preventing of diseases in connection with malfunctions of the blood-brain barrier, in particular structures and/or functions of the meninges.

In a preferred embodiment of the present invention, the recombinant peptide of interest is a collagen peptide for the use in a method of treating and/or preventing of intestine diseases, in particular chronic-inflammatory intestine diseases.

In a preferred embodiment of the present invention, the recombinant peptide of interest is a collagen peptide for the use in a method of treating and/or preventing of diseases of the cardiovascular system, in particular structure and/or function of the blood vessels, in particular the vascular wall, in particular treating and/or preventing of high blood pressure and/or circulatory disturbances.

In a preferred embodiment of the present invention, the recombinant peptide of interest is a collagen peptide for the use in a method of treating and/or preventing of diseases of the periodontium.

In a preferred embodiment, the present invention also pertains to the non-therapeutical use of a collagen peptide of the present invention for optical and structural enhancement of the skin, in particular for the reduction of wrinkle formation, enhancement of skin elasticity, increased tonicity of the skin, increased moisture content of the skin, reduction of cellulitis and/or reduction of stretch marks, in particular stretch marks in connection with pregnancy.

In a preferred embodiment, the present invention also pertains to the non-therapeutical use of a collagen peptide of the present invention for acceleration of the growth of nails and/or reduction of brittleness of nails.

In a preferred embodiment, the present invention also pertains to the non-therapeutical use of a collagen peptide of the present invention for optical and structural enhancement of hair, in particular for enhancement of hair quality, reduction of split ends and/or reduction/delay of hair loss.

In a preferred embodiment, the present invention also pertains to the non-therapeutical use of a collagen peptide of the present invention for increasing the number of mitochondria and/or mitochondrial activity.

In a preferred embodiment, the present invention also pertains to the non-therapeutical use of a collagen peptide of the present invention for enhancement of endurance.

In a preferred embodiment, the present invention also pertains to the non-therapeutical use of a collagen peptide of the present invention for enhancement of mental performance.

In a preferred embodiment, the present invention, the collagen peptide of the present invention is adapted and suitable for oral use.

In a preferred embodiment, the present invention, the collagen peptide of the present invention is adapted and suitable for topical application.

In a preferred embodiment of the present invention, any nucleic acid sequence disclosed herein is pertaining to exactly the sequence as disclosed in the sequence protocol.

In a preferred embodiment of the present invention, any nucleic acid sequence as disclosed herein is pertaining to exactly the sequence as disclosed in the sequence protocol and/or to any nucleic acid sequence, preferably a functionally equivalent nucleic acid sequence variant, sharing at least 80 %, in particular at least 90 %, sequence identity, preferably determined using the BLAST algorithm.

As used herein, the term "sequence identity" or "identity" refers to the number (%) of matches (identical nucleic acid residues) in positions from an alignment of two polynucleotide sequences. The sequence identity is determined by comparing the sequences when aligned so as to maximize overlap and identity while minimizing sequence gaps. In particular, sequence identity may be determined using any of a number of mathematical global or local alignment algorithms, depending on the length of the two sequences. Sequences of similar lengths are preferably aligned using a global alignment algorithm (for instance Needleman and Wunsch algorithm; Needleman and Wunsch, Journal of Molecular Biology. Band 48, 1970, S. 443-453) which aligns the sequences optimally over the entire length, while sequences of substantially different lengths are preferably aligned using a local alignment algorithm (for instance Smith and Waterman algorithm, Smith and Waterman, Journal of Molecular Biology, Band 147, 1981, S. 195-1971) or Altschul algorithm (Altschul et al., Nucleic Acids Research, Bd. 25, Nr. 17, 1997). Alignment for purposes of determining percent nucleic acid sequence identity can be achieved in various ways that are within the skill in the art, for instance, using publicly available computer software available on internet web sites such as http://blast.ncbi.nlm.nih.gov/ or http://www.ebi.ac.uk/Tools/emboss/). Those skilled in the art can determine appropriate parameters for measuring alignment, including any algorithms needed to achieve maximal alignment over the full length of the sequences being compared. For purposes herein, % nucleic acid sequence identity values refers to values generated using the pair wise sequence alignment program EMBOSS Needle that creates an optimal global alignment of two sequences using the Needleman-Wunsch algorithm, wherein all search parameters are set to default values, that means Scoring matrix = BLOSUM62, Gap open = 10, Gap extend = 0.5, End gap penalty = false, End gap open = 10 and End gap extend = 0.5.

A preferred method for determining the best overall match between a query sequence (a sequence of the present invention) and a subject sequence, also referred to as a global sequence alignment, can be determined using the FASTDB computer program based on the algorithm of Brutlag et al. (Comp. App. Blosci. (1990) 6:237-245). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identity are: Matrix=Unitary, k-tuple=4, Mismatch Penalty--1, Joining Penalty--30, Randomization Group Length=0, Cutoff Score=l, Gap Penalty--5, Gap Size Penalty 0.05, Window Size=500 or the length of the subject nucleotide sequence, whichever is shorter. If the subject sequence is shorter than the query sequence because of 5' or 3' deletions, not because of internal deletions, a manual correction must be made to the results. This is because the FASTDB program does not account for 5' and 3' truncations of the subject sequence when calculating percent identity. For subject sequences truncated at the 5' or 3' ends, relative to the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is preferably used for the purposes of the present invention. Only bases outside the 5' and 3' bases of the subject sequence, as displayed by the FASTDB alignment, which are not matched/aligned with the query sequence, are calculated for the purposes of manually adjusting the percent identity score. For example, a 90 base subject sequence is aligned to a 100 base query sequence to determine percent identity. The deletions occur at the 5' end of the subject sequence and therefore, the FASTDB alignment does not show a matched/alignment of the first 10 bases at 5' end. The 10 impaired bases represent 10% of the sequence (number of bases at the 5' and 3' ends not matched/total number of bases in the query sequence) so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by FASTDB is not manually corrected. Only bases 5' and 3' of the subject sequence which are not matched/aligned with the query sequence are manually corrected for.

As used herein, the term "low stringency conditions" means for probes of at least 100 nucleotides in length, prehybridization and hybridization at 42°C in 5X SSPE, 0.3% SDS, 200 micrograms/mL sheared and denatured salmon sperm DNA, and 25% formamide, following standard Southern blotting procedures for 12 to 24 hours. The carrier material is finally washed three times each for 15 minutes using 2X SSC, 0.2% SDS at 50°C.

The term "medium stringency conditions" means for probes of at least 100 nucleotides in length, prehybridization and hybridization at 42°C in 5X SSPE, 0.3% SDS, 200 micrograms/mL sheared and denatured salmon sperm DNA, and 35% formamide, following standard Southern blotting procedures for 12 to 24 hours. The carrier material is finally washed three times each for 15 minutes using 2X SSC, 0.2% SDS at 55°C.

The term "high stringency conditions" means for probes of at least 100 nucleotides in length, prehybridization and hybridization at 42°C in 5X SSPE, 0.3% SDS, 200 micrograms/mL sheared and denatured salmon sperm DNA, and 50% formamide, following standard Southern blotting procedures for 12 to 24 hours. The carrier material is finally washed three times each for 15 minutes using 2X SSC, 0.2% SDS at 65°C.

In a preferred embodiment of the present invention, any amino acid sequence as disclosed herein is pertaining to exactly the sequence as disclosed in the sequence protocol.

In a preferred embodiment of the present invention, any amino acid sequence as disclosed herein is pertaining to exactly the sequence as disclosed in the sequence protocol and to any sequence sharing at least 80 %, in particular at least 90 %, in particular at least 95 %, sequence identity, preferably determined using the BLAST algorithm.

In the context of the present invention, the term "peptide" refers to an amino acid sequence of any length including dipeptides, oligopeptides, polypeptides and proteins.

In the context of the present invention, the term "collagen" as used herein is to be understood as referring to collagen as known to the person skilled in the art, in particular as defined in WO 01/34646. In a preferred embodiment the term "collagen" refers to collagen types I to XXVII, in particular type I or type II, in particular type I. In a further preferred embodiment the term "collagen" refers to a peptide having the sequence Glycine-Proline, Glycine-4-Hydroxyproline (Hyp) or Glycine-X-4-Hydroxyproline, preferably the repetitive motif (Gly-X-Y)n, wherein X and Y are each any amino acid, preferably Proline or 4-Hydroxyproline. Particularly preferred, the term "collagen" is to be understood to refer to a peptide having the repetitive motif (Gly-Pro-Y)n and/or (Gly-X-Hyp)ₘ, wherein X and Y are each any amino acid.

In the context of the present invention, the term "collagen peptide" as used herein is to be understood to refer to a peptide or protein that has an amino acid sequence of a collagen as defined herein, wherein the peptide or protein is at least a dipeptide, in particular an oligopeptide or a polypeptide. The collagen peptide can preferably be chemically modified, in particular hydroxylated and/or glycosylated, or not-modified. A "collagen peptide" according to the invention can also be a collagen protein. In particular, the collagen peptide can be a monomer as well a dimer or trimer, in particular a trimer, of the collagen peptides with the same or different amino acid sequence, in particular as a triple-helical collagen peptide.

In the context of the present invention, the term "fusion protein" as used herein is to be understood as a protein comprising at least two functional peptides, preferably originating from different proteins, in particular from different organisms, which are encoded in one open reading frame of a nucleic acid sequence and are, thus, expressed as one continuous amino acid sequence, in particular one protein.

In the context of the present invention, the term "functional peptide" as used herein is to be understood as a peptide, which fulfils a function in the cell where it originates from. In particular, this can be a peptide of any length, in particular signal peptides or protein domains or complete proteins.

In the context of the present invention, the term "genomic locus" as used herein is to be understood as referring to a position of a nucleic acid sequence on a chromosome, in particular a wildtype chromosome, in particular without genetic modification introduced by synthetic means.

In the context of the present invention, the term "stably integrated" as used herein refers to the integration of an exogenous nucleic acid sequence of interest into the chromosome of a fungal cell by adjoining the nucleic acid of the chromosome with the nucleic acid of interest. In comparison, the integration of an exogenous nucleic acid into the fungal cell without integration into a chromosome refers to a "transient" integration, whereby upon cell division the nucleic acid will not be propagated with the daughter cells equally.

In the context of the present invention, the term "central position on the chromosome" means a position of the yurl gene which is 3' to the native, that means naturally occurring, position of the yurl gene in Komagataella phaffii on chromosome 2, in particular in 3'-direction from the native position of the yurl gene on chromosome 2, preferably in a central position, preferably between the catalase locus and MRPL4, preferably at positions 255125 to 1577812, wherein position 255125 corresponds to the last base of the catalase (=PP7435_CH2-0137) and position 1577812 corresponds to the first base of the MRPL4 (=PP7435_CH2-0854) on the native chromosome 2, most preferably in an inverse orientation with respect to the native orientation of the yurl in its native position.

In the context of the present invention, the term "the native position on chromosome 2 of Komagataella phaffii", means positions from 211330 to 212520 of the yurl gene in Komagataella phaffii (Reference 4, Chr 2 is deposited at the NCBI under FR839629).

In the context of the present invention, the term "orientation on a chromosome" as used herein is to be understood as referring to the 5'- to 3'-orientation of a nucleotide sequence on the chromosome, in particular on one strand of a chromosome. In particular, "same orientation" means that the nucleotide sequences of, for example, two genes are both oriented in a 5'- to 3'-direction on the same nucleic acid strand of the chromosome, whereas "opposite orientations" means that, one of the nucleotide sequences is located in 5'- to 3'-orientation on one strand and the other nucleotide sequence is located in 5'- to 3'-orientation on the opposite nucleic acid strand of the same chromosome.

In the context of the present invention, the term "expression vector" as used herein is to be understood as a vector to introduce a specific exogenous nucleic acid sequence encoding a recombinant peptide of interest, in particular gene, into a target cell and which vector comprises genetic elements able to direct the cells mechanism for protein synthesis, in particular transcription and translation, to produce the peptide encoded by the exogenous nucleic acid sequence, in particular gene. Preferably, the expression construct is a plasmid or virus.

In the context of the present invention, the term "expression cassette" as used herein is to be understood as a DNA-segment, which is responsible for transcribing the genetic information encoded within this segment into RNA, in particular mRNA, and has at least a regulatory unit and a peptide coding nucleic acid sequence, commonly at least one promoter and at least one peptide coding nucleic acid sequence and optionally as a further regulatory unit a terminator, wherein the peptide coding nucleic acid sequence is functionally linked to the at least one regulatory unit.

The term "functionally linked" refers, as used herein, to the association of nucleic acid sequences on a single nucleic acid molecule so that the function of one is effected by the other. For example, a promoter is functionally linked with a coding sequence when it is capable of effecting the expression of that coding sequence, that means, the coding sequence is under the transcriptional control of the promoter.

In the context of the present invention, the term "conditions suitable for the expression of the recombinant peptide of interest" and "conditions suitable for the expression and hydroxylation of the recombinant peptide of interest" as used herein are to be understood as in particular conditions like temperature, pressure, time, light as well as presence or absence of an inducer and/or repressor, which enable or enhance an expression of the peptide of interest. In a preferred embodiment, the expression is performed in the context of a high cell density fermentation. The exact conditions, which enable the expression of the peptide of interest, are known to the person skilled in the art and depend on the used fungal cell and the used expression cassette, in particular the used regulatory units, in particular the used promoter. The expression can in particular be constitutive, derepressed or inducible.

In the context of the present invention, the term "methylotrophic" is to be understood as referring to an organism that can live with methanol as the sole carbon source.

In the context of the present invention, the designations "Pichia pastoris" and "Komagataella phaffi" refer to the same fungal species and can be used interchangeably.

In the context of the present invention, the term "fungal cell" as used herein is to be understood as referring to any state of a fungal cell, including in particular protoplasts, spores, in particular sexual and asexual spores, single cells and hyphae.

In the context of the present invention, the term "fungal host cell" refers to a cell of a fungus, which is subjected to a transformation with at least one exogenous nucleic acid sequence, in particular so as to obtain a fungal cell according to the present invention.

In the context of the present invention, the term "nucleic acid sequence" refers to a continuous stretch of nucleotides, that means polynucleotides, in particular DNA polynucleotides.

In the context of the present invention, the term "nucleic acid sequence" or "nucleotide sequence" as used herein is in particular to be understood as the continuous sequence or stretch of the bases adenine, thymine or uracil, guanine and cytosine of a nucleic acid, in particular a poly nucleic acid, in particular DNA. The "nucleic acid sequence" is, thus, to be understood as an information entity as well as the physical manifestation of this information in the form of a nucleic acid, in particular DNA. An "exogenous nucleic acid sequence" is a nucleic acid sequence which was introduced into an organism by technical means and is preferably a nucleic acid sequence not found naturally in that organism.

In the context of the present invention, the term "exogenous nucleic acid sequence encoding a peptide", the peptide being a peptide of interest or a P4H, PIN4H or lysyl hydroxylase, is referring to a nucleic acid sequence, which comprises or consists of a stretch of nucleotides which are encoding the peptide being referred to, that means being encoded by said nucleic acid sequence and, thus, refers to a nucleic acid sequence which may optionally in addition to the open reading frame for the encoded peptide comprises nucleotides having other or no functions. Preferably, the term "exogenous nucleic acid sequence encoding" refers to a nucleic acid sequence which consists of a nucleic acid sequence being an open reading frame for a peptide.

The term "gene" means the segment of DNA involved in producing a polypeptide chain; it includes regions preceding and following the region encoding a peptide, that means comprising 5' and 3' located non-coding, but regulatory regions as well as intervening sequences (introns) between individual coding segments (exons).

In the context of the present invention, the term "copy" refers to an "exogenous nucleic acid sequence encoding a peptide" as defined herein and is used herein to define the number of such exogenous nucleic acid sequences as being used or contained in an expression cassette, in an expression vector, in a gene, in a chromosome and/ or in a fungal cell and in a method to use or produce any of them.

In the context of the present invention, the term "and/or" as used herein is to be understood in the way that all members of a group connected with the term "and/or" are disclosed as alternatives to each other as well as any possible combination of these members. For example, the term "A, B and/or C" is to be understood to pertain to a) (A or B or C) or b) (A and B) or c) (A and C) or d) (B and C) or e) (A and B and C).

In the context of the present invention, the term "at least" as used herein is to be understood to disclose the amount as stated by the number following the term without limiting the maximal amount, and also exactly the amount as represented by the number following the term, thus, limiting the amount to the number as stated.

In the context of the present invention, the term "comprising" as used herein is to be understood as to have the meaning of "including" or "containing", which means that in addition to the explicitly mentioned element further elements are possibly present.

In a preferred embodiment of the present invention, the term "comprising" as used herein is also to be understood to mean "consisting of' thereby excluding the presence of other elements besides the explicitly mentioned element.

Thus, the term "consisting of' is to be understood as meaning that solely the explicitly mentioned elements are present and the presence of other elements not explicitly mentioned is excluded.

In a furthermore preferred embodiment, the term "comprising" as used herein is also to be understood to mean "consisting essentially of' thereby excluding the presence of other elements providing a significant contribution to the disclosed teaching besides the explicitly mentioned element.

The sequence listing shows:
SEQ ID No. 1 the DNA sequence of a codon-optimized bovine 45 kDa ColA1 coding region,
SEQ ID No. 2 the amino acid sequence encoded by SEQ ID No. 1 (Col45opt),
SEQ ID No. 3 the DNA sequence of forward primer GE34,
SEQ ID No. 4 the DNA sequence of reversed primer GE35,
SEQ ID No. 5 the DNA sequence of forward primer GE36,
SEQ ID No. 6 the DNA sequence of reversed primer GE37,
SEQ ID No. 7 the DNA sequence of forward primer GE40,
SEQ ID No. 8 the DNA sequence of reversed primer GE41,
SEQ ID No. 9 the DNA sequence of vector pBSYGAPsec_blunt1,
SEQ ID No. 10 the DNA sequence of vector pGAPsec-Col45opt,
SEQ ID No. 11 the DNA sequence of vector pBSY3S 1K,
SEQ ID No. 12 the DNA sequence of vector pCATsec-Col45opt,
SEQ ID No. 13 the DNA sequence of the native yurl coding region,
SEQ ID No. 14 the amino acid sequence encoded by the native yurl gene according to SEQ ID No. 13,
SEQ ID No. 15 the DNA sequence of the truncated yurl gene
SEQ ID No. 16 the amino acid sequence encoded by the truncated yurl coding region according to SEQ ID No. 15,
SEQ ID No. 17 the DNA sequence of vector pCATsec_Col45yur1,
SEQ ID No. 18 the DNA sequence of vector pAOX_Mimi-int 3.0,
SEQ ID No. 19 the DNA sequence of vector pAOX_Mimi-int 3.1,
SEQ ID No. 20 the DNA sequence encoding MF alpha (1-57 pre-sequence, 58-267 pro-sequence) used for expression of Col45opt,
SEQ ID No. 21 the amino acid sequence of MF alpha (1-19 pre-sequence, 20-89 pro-sequence) according to SEQ ID No. 20,
SEQ ID No. 22 the amino acid sequence of SEQ ID No. 2 (Col45opt), in which prolines are hydroxylated to hydroxy prolines,
SEQ ID No. 23 the DNA sequence of the native P4H, gene L593,
SEQ ID No. 24 the amino acid sequence encoding the native P4H, L593 of SEQ ID No.23,
SEQ ID No. 25 the DNA sequence of the P4H in pAOX_Mimi-int 3.0 and
SEQ ID No. 26 the amino acid sequence encoded by SEQ IQ No. 25.

Further preferred embodiments of the present invention are the subject-matter of the dependent claims.

In the following examples and the accompanying figures, the present invention is explained in more detail without limiting the present invention.

The figures show:
- Figure 1: a vector map of vector pBSYGAPsec_blunt1
- Figure 2: a vector map of pGAPsec-Col45opt,
- Figure 3: a vector map of pBSY3S 1K,
- Figure 4: a vector map of pCATsec-Col45opt,
- Figure 5: the results of a Dot Blot experiment with supernatants of Col45 expressing transformants of Komagataella phaffii,
- Figure 6A: a graphic representation of cultivation experiments of the pCATsec-Col45opt clone F2, (K. phaffi 45-I) with respect to the oxygen transfer rates and a negative control (nc),
- Figure 6B: the results of an SDS-PAGE experiment showing expression of the clones according to Figure 6A,
- Figure 7: a schematic representation of the results of a genomic analysis of chromosome 2 of Col45 expressing strain pCATsec-Col45opt K. phaffii 45I-1 (A and B),
- Figure 8: a detailed representation of the C-terminal region of Yur1 in K. phaffii 451-1 (F2) and the native strain,
- Figure 9: a detailed presentation of the downstream integration region in strain K. phaffii 45I-1,
- Figure 10: Col45 expression of clones transformed with pCATsec_Col45yur1 linearized in yurl,
- Figure 11: Col45 expression of strains with single integrations of a Col45 expression cassette in yurl (B3) or Chr1-1228 (A12),
- Figure 12: a vector map of plasmid pCATsec-Col45yur1,
- Figure 13: Col45 expression of three Δyur1 derivatives, namely clones 1, 2 and 3, of strain K. phaffii 451-1,
- Figure 14: a vector map of plasmid pAOX_Mimi-int 3.0,
- Figure 15: a schematic representation of the genomic integration K. phaffii 451-1+ pAOX_Mimi-int 3.0 (including P4H) resulting in the K. phaffii 451-1 derivative E11 (1 copy pAOX_Mimi-int 3.0 in fourth collagen copy integrated),
- Figure 16: a schematic representation of the genomic integration clone E11 + further collagen copy resulting in the K. phaffii 451-1 derivative K. phaffii 451-2, comprising a further (sixth) collagen copy between 1. + 2. original copies integrated and
- Figure 17: a vector map of plasmid Plasmid pAOX_Mimi-int 3.1.

### Examples:

### Example 1 Cloning procedure

### 1.1 Construction of plasmid pGAPsec-Col45opt

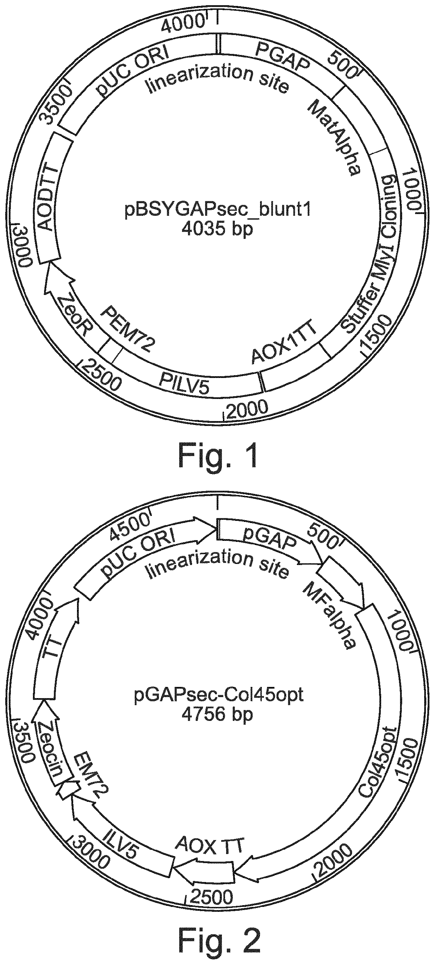

A nucleic acid sequence of interest in form of a codon-optimized bovine based CollAl gene (SEQ ID No. 1) (encoding a bovine 45 kDa collagen peptide type I, A1, hereinafter also called Col45, SEQ ID No. 2) was cloned into expression vector pBSYGAPsec_blunt1 (Figure 1) (SEQ ID No. 9). For that purpose, the Col1A1 fragment was amplified with primers Ge34 (SEQ ID No. 3) and primers Ge35 (SEQ ID No. 4); both equipped with overlapping ends for Gibson cloning in the MlyI/NotI digested vector.

**Table 1: Primer identification**

| Primer | Sequence 5' - 3' | Direction | Purpose |
|---|---|---|---|
| Ge34 (SEQ ID No. 3) | tcgagaagagagaggccgaagctGAGC GTGGATTTCCAGGCGAAAGG | For | Amplification 45 kDa Col1A1 |
| Ge35 (SEQ ID No. 4) | ttctgacatcctcttgattaAGAAGGAGG ACCAGGAGGTCCTGGAGG | Rev | |
| Ge36 (SEQ ID No. 5) | ATGAGATTCCCATCTATTTTC | For | Amplification backbone for promoter exchange |
| Ge37 (SEQ ID No. 6) | ACATGTGAGCAAAAGGCC | Rev | |
| Ge40 (SEQ ID No. 7) | ctggccttttgctcacatgtTAATCGAACTCCGAATGC | For | Amplification pCAT |
| Ge41 (SEQ ID No. 8) | gtgaaaatagatgggaatctcatTTTAATTG TAAGTCTTGACTAGAG | Rev | |

**Table 2: PCR conditions for Q5 High fidelity PCR polymerase (NEB)**

| Component | 25 µl Reaction | 50 µl Reaction | Final Concentration |
|---|---|---|---|
| 5x Q5 Reaction Buffer | 5 µl | 10 µl | 1× |
| 10 mM dNTPs | 0.5 µl | 1 µl | 200 µM |
| 10µM Forward Primer | 1.25 µl | 2.5 µl | 0.5µM |
| 10µM Reverse Primer | 1.25 µl | 2.5 µl | 0.5 µM |
| Template DNA | Variable | Variable | < 1,000 ng |
| Q5 High-Fidelity DNA Polymerase | 0.25 µl | 0.5 µl | 0.02 U/µl |
| 5X Q5 High GC Enhancer (optional) | (5 µl) | (10 µl) | (1×) |
| Nuclease-Free Water | To 25 µl | To 50 µl | |

**Table 3: Thermal cycler conditions**

| Step | Temp. | Time |
|---|---|---|
| Initial Denaturation | 98 °C | 30 seconds |
| 25 - 35 Cycles | 98 °C | 5 - 10 seconds |
| | ^{∗}50 - 72 °C | 10 - 30 seconds |
| | 72 °C | 20 - 30 seconds |
| Final Extension | 72 °C | 2 minutes |
| Hold | 4 - 10 °C | |

Annealing temperatures were calculated with the NEB Tm calculator.

The PCR reaction was applied on an 1 % agarose gel and bands with the correct size were excised from the gel and purified using the Monarch DNA Gel extraction kit.

The isolated DNA fragment was cloned into the linearized vector using the Gibson Assembly. 0.06 to 1.2 pmol of DNA was used and the insert was used with 5- to 10-fold excess. The mixture was incubated for 60 min at 50°C and 2.5 µl subsequently transformed into E. coli DH5alpha (NEB C2987H) following the manufacturer's protocol.

Transformants were selected on LB agar supplemented with 50 µg/ml zeocin. Successful cloning was verified by colony PCR using the OneTaq-2 x Master Mix (NEB M0482), again with primers Ge34 (SEQ ID No. 3) and Ge35 (SEQ ID No. 4).

**Table 4: PCR conditions OneTaq Master Mix**

| Component | 25 µl reaction | 50 µl reaction | Final concentration |
|---|---|---|---|
| 10 µM Forward Primer | 0.5 µl | 1 µl | 0.2 µM |
| 10 µM Reverse Primer | 0.5 µl | 1 µl | 0.2 µM |
| Template DNA | Variable | Variable | < 1,000 ng |
| One Taq 2x Master Mix with Standard Buffer | 12.5 µl | 25 µl | 1× |
| Nuclease-Free Water | To 25 µl | To 50 µl | < 1,000 ng |

For cycling a 2-step PCR was performed:
1.) 30 sec 95 °C
2.) 30 sec 95 °C
3.) 2 min 68 °C
4.) 5 min 68 °C

Step 2 and 3 were repeated with 29 cycles. The sequence from a random clone showing the respective Col45opt DNA fragment was verified by Sanger Sequencing (Eurofins Genomics). pGAPsec-Col45opt (Figure 2) (SEQ ID No. 10) was obtained.

### 1.2 Construction of plasmid pCATsec-Col45opt

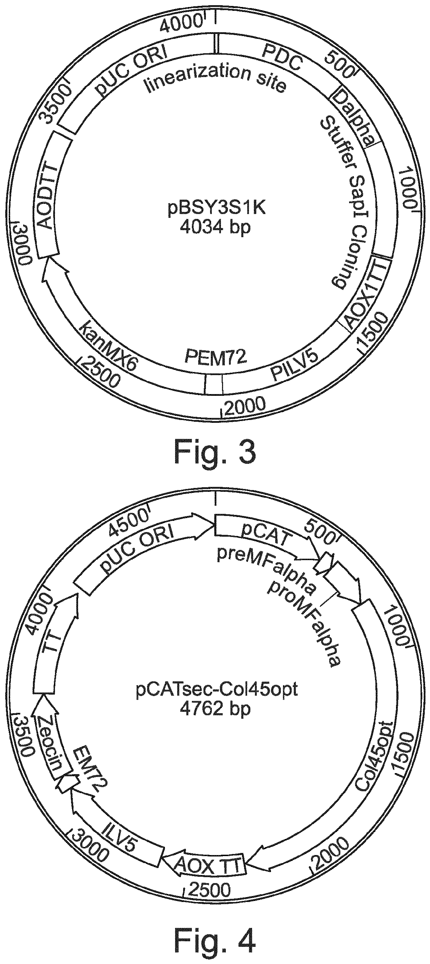

For construction of plasmid pCATsec-Col45opt (Figure 4) (SEQ ID No. 12) the promoter of plasmid pGAPsec-Col45opt was exchanged. Therefore, the vector region of pGAPsec-Col45opt except the promoter was amplified using primers Ge36 (SEQ ID No. 5) and Ge37 (SEQ ID No. 6) and the PCR reaction was subsequently digested with DpnI to degrade the template DNA.

The sequence for pCAT was amplified from the episomal plasmid pBSY3S1K (Figure 3) (SEQ ID No. 11) with primers Ge40 (SEQ ID No. 7) and Ge41 (SEQ ID No. 8), both equipped with overlaps for Gibson Cloning in the vector backbone. The PCR product was purified using the Monarch PCR & DNA Cleanup kit.

Both PCR products were ligated using Gibson Assembly Mix and transformed in E. coli. After colony PCR showing the expected fragment for pCAT the correct DNA sequence was verified and, thus, vector pCATsec-Col45opt (SEQ ID No. 12) (Figure 4) was obtained.

This vector contains the Col45 encoding nucleic acid sequence in open reading frame together with the mating factor α encoding nucleic acid sequence (fusion peptide) functionally linked to the pCAT promoter and the AOX TT terminator.

### Example 2 Transformation of pCATsec-Col45opt in Komagataella phaffii BG11

Plasmid pCATsec-Col45opt (SEQ ID No. 12) was linearized with BstBI in pCAT. 2.9 µg of plasmid were cut with 30 U of BstBI in a total volume of 20 µl. The digestion reaction was allowed to last for 1.5 hours at 37°C, then it was purified using the Monarch PCR & DNA Cleanup kit. 370 ng were transformed in 40 µl of competent cells of strain Komagataella phaffii pBSYBG11 which were prepared following the protocol of Lin-Cereghino, 2005 (Reference 1). Transformants were selected from YPD agar (10 g/L yeast extract, 20 g/L peptone, 10 g/L dextrose, 1,5 % agar) supplemented with 1 M sorbitol and 500 µg/ml Zeocin after incubation for 3 days at 30°C. 77 transformants were picked on YPD agar containing 100 µg/ml Zeocin prior to expression experiments.

Strain Komagataella phaffii BSYBG11 is derived from yeast strain Komagataella phaffii (Komagataella phaffii) BSYBG10: Mut+, killer plasmid free wt strain BSYBG10. BSYBG11: The AOX1 ORF (open reading frame) was deleted in killer plasmid free wt (wild type) strain BSYBG10, the ORF was replaced by an inactivated lox71/lox66 sequence and TTFDH1 (Reference 2).

The wildtype strains on which strains Komagataella phaffii BSYBG10 and BSYBG11 (Bisy GmbH, 8010 Graz, Austria) are based on is Komagataella phaffii CBS7435 (or NRRL-Y11430, ATCC 76273) (References 2-4).

### Example 3 Expression of Col45

The transformants were transferred in 0.3 mL YPD medium supplemented with 100 mg/L Zeocin and cultivated in square 96 deep well microplates covered with the suitable sandwich cover (enzyscreen.com). Cultivation conditions were shaking with 300 rpm, 50 mm throw at 30°C. After 24 hours 3 µl of each preculture was transferred into Syn 6 minimal medium (Stöckmann, 2003) (Reference 5) (Example 9) supplemented with 2% methanol to induce collagen production. The strains were cultivated under the same culture conditions for 72 hours.

### Example 4 Dot Blot analysis

The microplates containing the grown cultures were centrifuged at room temperature for 10 min and 3000 x g and the supernatants were used for a Dot Blot analysis. 5 µl were transferred onto an equilibrated PVDF membrane which was prewetted for 15 sec in 96% ethanol, 2 min in tap water and 5 min in towbin transfer buffer (48 mM Tris, 39 mM glycine, pH 9.2, 10% ethanol) placed on 10 sheets prewetted filter paper on 20 sheets dry filter paper to allow protein transfer. After the applied liquid was absorbed the membrane was allowed to dry.

The dried membrane was incubated in blocking solution (0.5 g BSA and 2.5 g milk powder in TBST) overnight. The blocked membrane was washed 3 times for 5 min in TBST (10 mM Tris, 5.5 g/L NaCl, 5 ml/L Tween 20, pH 8) and incubated with 1:5000 diluted Anti-CollAl antibody HRP conjugate (0.5 mg/mL NBP2-46875, Bio-Techne) for 1 hour at 28°C. Before detection the membrane was washed 3 times in TBST and 3 times in 100 mM Tris-HCl pH 8.5. The washed membrane was covered with Pierce ECL Western Blotting Substrate (Thermo Scientific # 32109) and the signal was monitored for 5 to 250 sec exposure time.

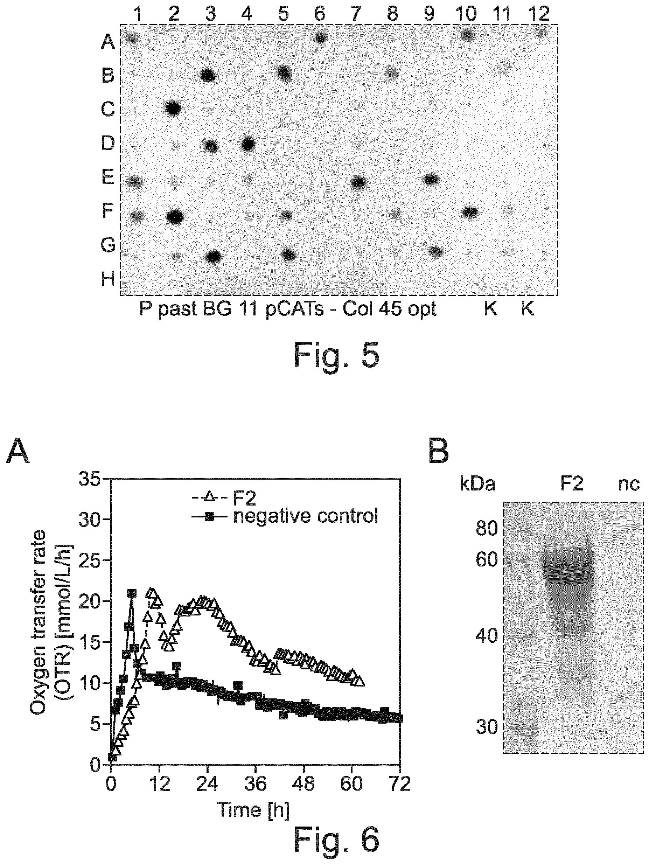

Figure 5 shows a dot blot analysis with supernatants from Col45 expressing transformants of Komagataella phaffii BSYBG11::pCATsec-Col45opt. Position F2 represents the strongest clone used for the later fermentation experiments.

### Example 5 Cultivation in membrane-based fed-batch shake flasks

As strain performance of individual clones is affected by the mode of cultivation (batch versus fed-batch operation mode) (Reference 6), the obtained strains are generally tested in fed-batch operation mode to ensure the selection of the best possible producer. Therefore, the membrane-based fed-batch shake flasks are utilized (Reference 7, 8) in combination with the respiration activity monitoring system (RAMOS) to measure the oxygen transfer rate (OTR) during the cultivation online (Reference 9). The detailed set-up and preparation of the membrane-based fed-batch shake flasks are described in (Reference 10).

In the standard screening protocol, 250 mL shake flasks are filled with 10 mL of Syn6 medium (see Example 9) where the carbon source is excluded. These flasks are inoculated with an initial OD600 of 0.8 and combined with a reservoir containing 3 mL of feed solution. The feed is an aqueous solution, containing 200 g/L glucose and 20 % v/v methanol. The reservoir and the cell containing Syn6 media are separated by a cellulose membrane (thickness= 42µm, cut-off= 10-20kDa). Due to the concentration gradient between reservoir and Syn6 medium, glucose and methanol diffuse simultaneously into the culture broth and thereby enable a constant supply of both substrates. The course of the OTR indicates at what time point a steady feed supply is reached (Fig. 6A). After 60 hours of cultivation, the culture broth is harvested and the cells are separated via centrifugation. The remaining supernatant is further analyzed by SDS-PAGE (Fig 6B) to evaluate the different strains in regard of productivity. Figure 6 shows cultivation of K. phaffii::pCATsec-Col45opt clone F2 (K. phaffi 451-1) in Syn6 medium under fed batch conditions. A: Oxygen transfer rates B: Expression of 45 kDa collagen, buffer: MES 200 mM pH 6, C-source: glucose, T= 30°C, n=350 rpm, d0=5 cm, VL= 10 mL in 250 mL shake flasks, OD600, Start=0.8, 3 mL reservoir volume with 200 g/L glucose and 20% v/v MeOH. Marker was Roti-Mark 10-150 PLUS. nc: negative control (K. phaffi clone with integrated GFP-gene).

From 96 clones obtained in Example 2 and tested according to the present example the clone giving the highest titer of Col45 (clone F2) (Figure 6) has been deposited with the Leibniz-Institut (DSMZ-Deutsche Sammlung von Mikroorganismen und Zellkulturen GmbH, Germany) in accordance with the provisions of the Budapest Treaty under the designation Komagataella phaffii 451-1 = DSM 33955, deposited on 28.07.2021. A clone termed A12 with hardly recognizable collagen expression was also analysed and it was found that a Col45opt integration took place in Chr1-228, but not in the yurl gene.

### Example 6 Genomic Analysis of strain K. phaffii 451-1

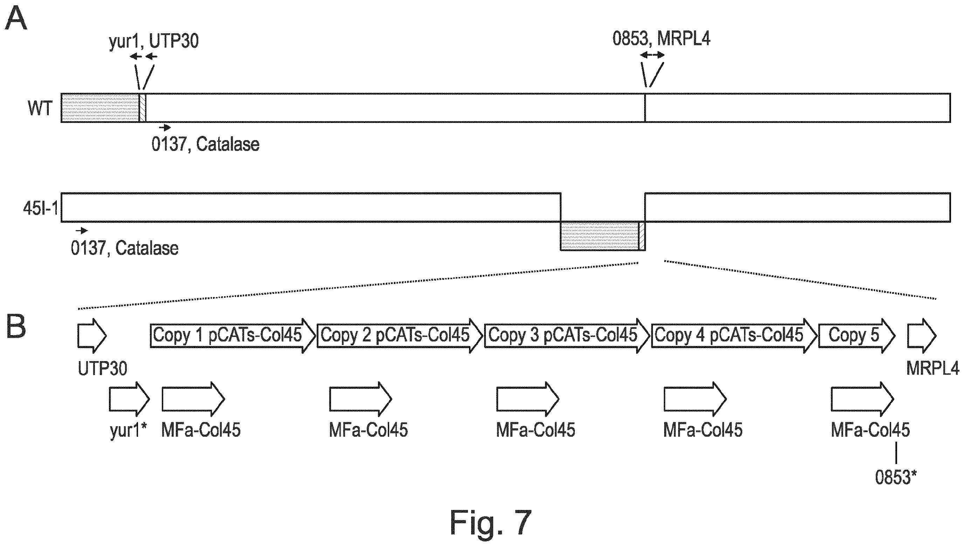

The genome of collagen producer K. phaffii 451-1 was analyzed by whole genome sequencing (Fig. 7). Figure 7 shows the genomic analysis of its chromosome 2. Parts of the chromosome were rearranged causing a translocation of two regions from the upstream end to the center of the chromosome. The genes yurl (PP7435_CHR2-0110) and PP7435_CHR2-0853, a protein of unknown function, were disrupted (Figure 7A). Five vector copies of pCATsec-Col45opt (SEQ ID No. 12) integrated in yurl, coding for mannosyltransferase, resulting in five Col45 copies (Figure 7B). WT: wildtype, 451-1: collagen producer strain. UTP30 (PP7435_ CHR2-0111): putative subunit of U3-containing 90S pre-ribosome complex. MRPL4 (PP7435_ CHR2-0854): 54S ribosomal protein L4, mitochondrial. Five copies of vector pCATsec-Col45opt resulting in 5 copies of Col45opt were integrated into chromosome 2.

In the collagen producing strain two regions were translocated when compared to the native chromosome 2. In the first event more than 200 kbp, namely bases 211368 to the 5'end of the native Chr 2 (horizontal slashes in Figure 7A), were moved in 3'direction to the center of the chromosome and inverted. Downstream of this event a second translocation of at least 20kbp corresponding to the region upstream of base 211330 was found (diagonal stripes). This 20 kbp region was additionally inverted resulting in gene yurl placed at the 3'end of the new arrangement.

5 complete copies of vector pCATsec-Col45opt were integrated (Figure 7B) in yurl (see SEQ ID No. 13 and 14 for the DNA coding sequence and amino acid sequence of native yurl). In the wildtype strain, this gene is located on the complement strand. Within translocation the orientation switched and the collagen cassettes were fused in the same orientation.

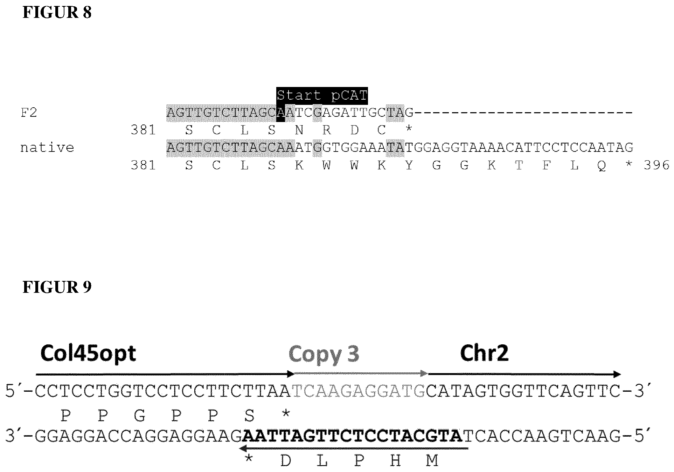

The exact integration site in yurl (PP7435_CHR-0110, 1191 bp, 396 amino acids) was found to be near the native C-terminus of the protein. This results in a truncated Yur1 protein (SEQ ID No. 15 and SEQ ID No. 16) as the integration causes an in-frame stop-codon in the inserted pCAT sequence (Figure 8). Figure 8 shows the C-terminal region of Yur1 in the native strain and K. phaffii 451-1. The insertion of the collagen cassette in yurl truncates the 12 C-terminal amino acids of the native Yur1 and adds NRDC instead. Identical bases of the fusion and native protein are marked in grey in Figure 8 while the start of the pCAT sequence is depicted in black.

Downstream the insertion of the collagen gene cassette leads to an interruption of an Open Reading Frame (ORF) located on the antisense strand and coding for a protein of unknown function (PP7435_CHR-0853) with 507 base pairs or 168 amino acids in size. Figure 9 shows the downstream integration region in strain K. phaffii 451-1. The insertion of the collagen cassette causes a new short open reading frame located on the antisense strand consisting of the start codon of gene PP7435_CHR-0853 and the insertion cassette.

### Example 7 Investigation of Yur 1 as integration locus and expression studies

### 7.1 Construction of col expressing clones with targeted integration into the yurl locus

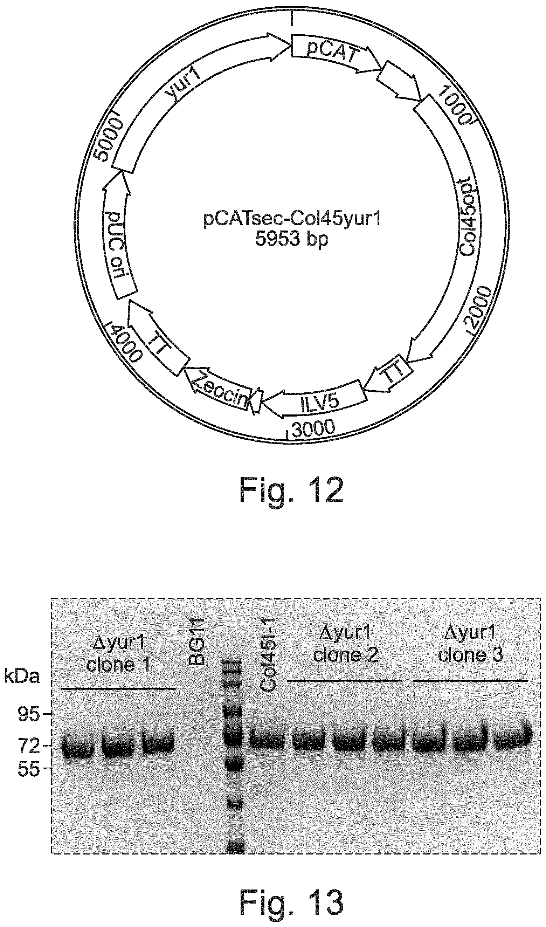

To investigate Yur1 as integration site a directed insertion of a Col45 expression cassette was pursued. Therefore, yurl was cloned upstream of pCAT giving plasmid pCATs-Col45yur1 (Fig. 12, SEQ ID No. 17), which was linearized in yurl with BstXI. 94 clones were screened for Col45 titers.

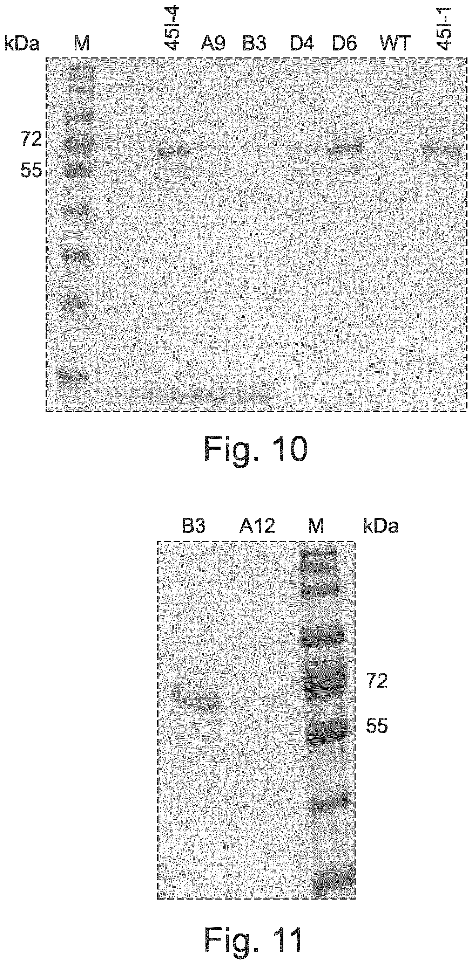

The clones were cultivated in fed-batch mode including strain K. phaffii 451-1 as reference strain. 2 clones (A2 (K. phaffii 451-4) and D6) showed a comparable titer as strain K. phaffii 451-1 (Figure 10). Clone A2 has been deposited with the Leibniz-Institut (DSMZ-Deutsche Sammlung von Mikroorganismen und Zellkulturen GmbH, Germany) in accordance with the provisions of the Budapest Treaty under the designation Komagataella phaffii 451-4 = DSM 33958, deposited on 28.07.2021.The genomes of these two strains and those of four more clones, two with good Col45 production (A9 and D4), one with sufficient production (B3) and one with no or at best very small production (A12) were analyzed by genome sequencing showing that the Col45 expression is clearly related to the copy number integrated. While strains 451-4 and D6 have 3 or 4 col45 copies integrated into the yurl locus, the good strains A9 and D4 contain 2 copies and the sufficient strain B3 only one copy. Strain A12 is a sister clone of K45I-1 in which only one copy of plasmid pCATsec-Col45opt was integrated, however, not in the yurl gene but in Chr1-1228.

For an approximate calculation of the collagen production rate of the above mentioned clones the titer was estimated by comparison to a lysozyme standard which was applied in different concentrations (0.5-2 g/L) onto the SDS gel. The intensity of the lysozyme signals was compared to the clone signals. For strains 451-4, D6 and 451-1 the signal strength was evaluated to be stronger than 2 g/L of lysozyme or 400 mg/L under consideration of the volume applied (5 µl). These calculated titers were referred to the duration of the experiment which lasted 72 hours. Therefore, for 451-4, D6 and 451-1 production rates greater than 5.6 mg L⁻¹ h⁻¹ are indicated (Table 5).

**Table 5: Copy numbers and targeted insertion of the Col45 expression cassette in yurl.**

| **Strain** | **Production rate [mg*L⁻¹*h⁻¹]** | **Copy numbers of Col45 and integration site** |
|---|---|---|
| 451-1 | >> 5.6 | 5 copies in yurl |
| 451-4 | >> 5.6 | 3 copies in yurl |
| D6 | >> 5.6 | 4 copies in yurl |
| A9 | 1.4 | 2 copies in yurl |
| D4 | 1.4 | 2 copies in yurl |
| B3 | </= 1.4 | 1 copy in yurl |
| A12^{∗} | Below detection limit | 1 copy in Chr1-1228 |

Clones with integrated plasmid pCATsec-Col45optYur1 linearized in yurl showing different production rates were analyzed by whole genome sequencing, (A12^{∗}: see Example 5.)

To compare the impact of the integration locus clone B3 with one copy of pCATsec-Col45optYur1 integrated in the yurl locus was recultivated under collagen producing conditions. Furthermore, strain A12 in which a single copy of plasmid pCATsec-Col45opt was integrated in Chr1-1228 was co-cultivated to ensure the same conditions. It was found that the integration in yurl (strain B3) resulted in a significantly higher expression than the integration in Chr1-0228, coding for 1,3-beta-glucan synthase component (Figure 11). In A12 hardly any, if at all, expression of collagen was detectable.

Figure 11 shows Col45 expression in supernatants of strains with single integrations of a Col45 expression cassette in yurl or Chr1-1228. Lane 1: strain B3 with pCATsec-Col45opt integrated in yurl lane 2: strain A12 with pCATsec-Col45opt integrated in Chr1-1228.

### 7.2 Collagen expression with strain K. phaffii 45I-1Δyur1

The impact of yurl in strain K. phaffii 451-1 was investigated. For a negative control, the complete truncated yurl ORF was deleted. Three independent Δyur1 derivatives of strain K. phaffii 451-1, namely clones 1, 2 and 3, were cultivated in triplicates under collagen expressing conditions. As controls, the parent strain K. phaffii 451-1 and the wildtype strain BG11 were co-cultivated. It was observed that the knock-out of yurl does not have an impact on collagen expression (Fig. 13).

### Example 8 Production of hydroxylated collagen peptide Col45

### 8.1 Construction of the hydroxylated Col45 expressing strain K. phaffii E11 (5 copies of Col45 and one copy of Mimi-PH4)

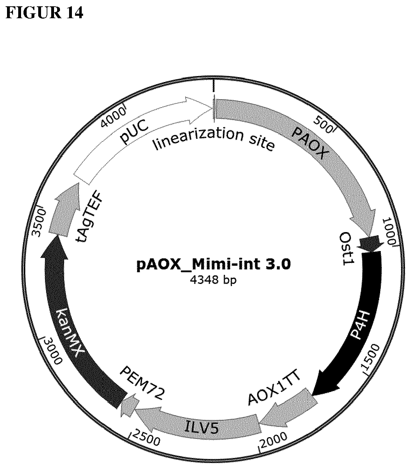

Strain K. phaffii 451-1 was further engineered to release hydroxylated Col45. For this purpose, it was transformed with plasmid pAOX_Mimi-int 3.0 (Fig. 14, SEQ ID No. 18). This plasmid was integrated into the genome of strain K. phaffii 451-1 and expresses in the obtained derivative with the designation E11 the prolyl-4-hydroxylase (P4H) from the Mimivirus of Acanthamoeba polyphaga (gene L593, protein EC:1.14.11.2). The P4H enables the posttranslational hydroxylation of proline incorporated in Col45 resulting in hydroxyproline residues. The estimated naturally occurring hydroxylation ratio of Col45 is 42% (estimated from UniprotKB-P02457 COL1A1_CHICK) (the hydroxylated prolines according to UniprotKB-P02457 COL1A1_CHICK are shown in SEQ ID No. 22).

To this end, the P4H expression vector pAOX_Mimi-int 3.0 (Figure 14) was linearized with BamHI downstream of the AOX terminator. In this plasmid the native secretion signal of the P4H (SEQ ID No. 23, DNA sequence of native P4H of Mimivirus gene L593) (SEQ ID No. 24 shows the encoded amino acid sequence) was replaced by the Ost1 sequence (Reference 14), allowing the transport of the protein into the ER. Furthermore, the P4H protein was equipped with a 6 x His tag and an ER retention signal.

SEQ ID No. 25 shows the DNA sequence of the P4H in pAOX_Mimi-int 3.0 and SEQ ID No. 26 the translated amino acid sequence with 1-22 Ost1, 23-28 HisTag, 29-250 P4H and the 251-254 retention signal. The linearized vector was transformed into strain K. phaffii 451-1.

96 geniticin resistant clones were cultivated in 24 well System Duetz plates in 1 ml Syn 6 medium, supplemented initially with 1 % (vol/vol) methanol and 20 µg sodium ascorbate. After 24 and 48 hours, the cultures were fed with again 1 % methanol and 160 µg sodium ascorbate. After 72 hours the cultures were harvested and the supernatant containing the hydroxylated collagen separated from the cells.

One of these clones, E11, was subjected to genomic sequencing. The genomic sequencing of the obtained strain K. phaffii E11 showed an integration of plasmid pAOX_Mimi-int 3.0 (Figure 14) in the AOX terminator region of Col45 copy 4.

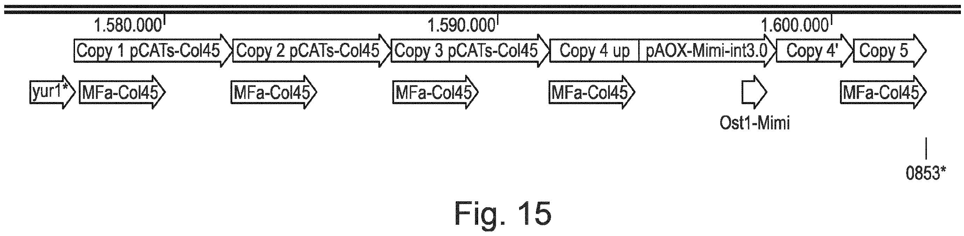

Fig. 15 shows the multicopy integration region in chromosome 2 with integrated plasmid pAOX_Mimi-int 3.0 in strain K. phaffii 451-1 resulting in the K. phaffii 451-1 derivative strain termed E11. The genomic sequencing revealed a single integration event in copy 4 of the collagen coding cassettes. The insertion is located in the AOX terminator region of copy 4.

The hydroxylation degree was determined with a gas chromatograph coupled to mass spectrometry (GC-MS). Therefore, 100 µl of the supernatant were precipitated with trichloroacetic acid (TCA) of a final concentration of 13,3 % (w/v) and stored 1h at 4°C. After centrifugation (4°C, 15 min and 17000 x g) the precipitate was washed with ice cold acetone, well resuspended by intense vortexing, centrifuged again and the acetone was allowed to evaporate overnight. The protein was hydrolyzed in 500 µl 6 M HCl at 105°C for 6 h, then the solution was transferred into a GC vial and dried at 60°C overnight. The amino acids were derivatized using N-(tert-Butyldimethylsilyl)-N-methyltrifluoracetamid (MBDSTFA) diluted 1:2 in acetonitrile in a final volume of 60 or 100 µl. The amino acids proline and hydroxyproline were detected by modifying the protocol for amino acid detection developed by Schmitz et al. (Reference 15).

**Table 6: detection parameters by GC-MS**

| | |
|---|---|
| Injector | |
| Sample volume | 1 µL |
| Injector Split | 10 |
| PTV inlet temperature | 250 °C |
| Flow rate | 1 mL/min |
| Oven | |
| Initial | 140 °C, 1 min hold |
| Ramp 1 | 15 °C/min, 310 °C, 3 min hold |

For proline the masses 184.15, 258.13 and 286.17 after a retention time of 6.28 min and for hydroxyproline the masses 182.14, 314.23 and 416.24 after 8.85 min were observed. The peak areas received for each sample were compared to a calibration curve obtained with standards ranging from 50 to 500 µM. As a control, a granule from bovine collagen was co-analyzed. E11 showed a hydroxylation degree of 50 %. Bovine collagen was used as standard and its hydroxylation ratio was considered to be 100 % as standard.

### 8.2 K. phaffii 451-2 (6 copies of Col45 and one copy of Mimi-PH4)

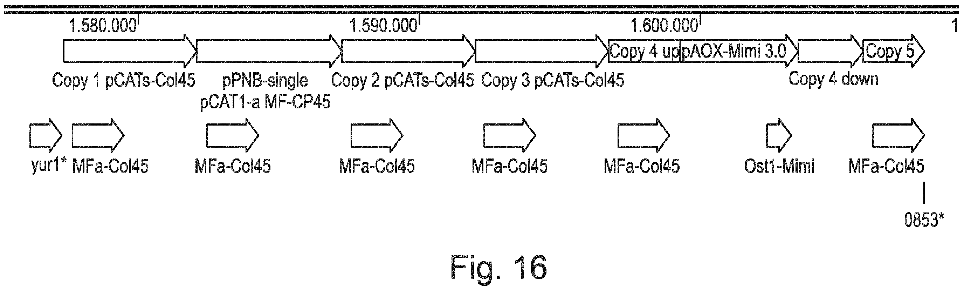

Another Col45 expression cassette was integrated into the genome of the K. phaffii 451-1 derivative E11 to result in a further derivative of K. phaffii 451-1, which is termed K. phaffii 451-2. The insertion is located between copy 1 and copy 2 of the Col45 insert in the parent strain. This clone has been deposited with the Leibniz-Institut (DSMZ-Deutsche Sammlung von Mikroorganismen und Zellkulturen GmbH, Germany) in accordance with the provisions of the Budapest Treaty under the designation Komagataella phaffii 451-2 = DSM 33956, deposited on 28.07.2021. Figure 16 shows the arrangement of the integration cassettes in strain K phaffii 451-2.

### 8.3 K. phaffii 451-3 (6 copies of Col45 and two copies of Mimi-PH4)

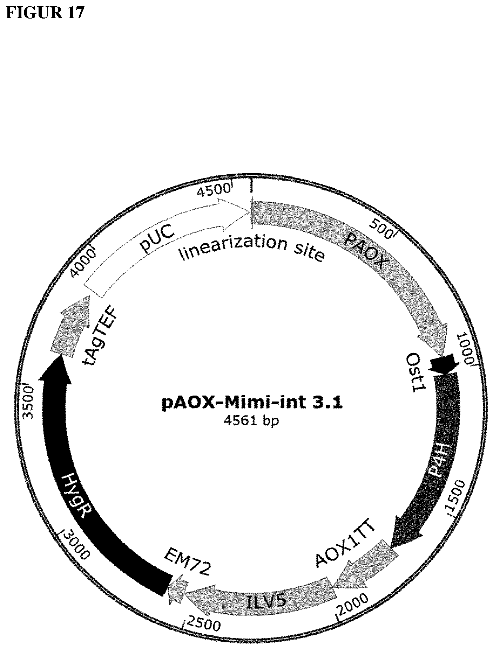

A second Mimi-PH4 expression cassette (pAOX_Mimi-int 3.1, Figure 17) was integrated into the genome of K. phaffii 451-2 to result in a further derivative of K. phaffii 451-1, which is termed strain K. phaffii 451-3. This clone has been deposited with the Leibniz-Institut (DSMZ-Deutsche Sammlung von Mikroorganismen und Zellkulturen GmbH, Germany) in accordance with the provisions of the Budapest Treaty under the designation Komagataella phaffii 451-3 = DSM 33957, deposited on 28.07.2021.

To the end, the KanMX gene of plasmid pAOX_Mimi-int 3.0 was replaced by a hygromycin cassette for the selection of clones. The resulting plasmid pAOX_Mimi-int 3.1 (Figure 17, SEQ ID No. 19) was linearized in pAOX and integrated into the genome of strain K. phaffii 451-2. The new transformants were cultivated under expression conditions to secrete hydroxylated Col45 and the hydroxylation degree from the collagen harvested in the supernatant was determined by GC-MS analytics. Therefore, 29 clones out of 96 transformants were selected randomly after collagen production was verified by SDS-analytics.

**Table 7: Hydroxylation degrees of derivatives (inter alia K. phaffii 45-3) of strain K. phaffii 451-2 and, thus, of 451-1, which contain a second P4H cassette. Bovine collagen was used as standard and its hydroxylation ratio was considered to be 100 % as standard.**

| Clone number | Hydroxylation degree [%] |
|---|---|
| 3 | 79 |
| 11 | 85 |
| 13 | 77 |
| 16 (K. phaffii 451-3) | 90 |
| 21 | 79 |
| 22 | 79 |
| 24 | 85 |
| 32 | 87 |
| 35 | 94 |
| 36 | 87 |
| 39 | 89 |
| 40 | 87 |
| 44 | 89 |
| 46 | 91 |
| 51 | 92 |
| 52 | 87 |
| 54 | 87 |
| 59 | 91 |
| 66 | 87 |
| 67 | 89 |
| 71 | 87 |
| 74 | 89 |
| 75 | 87 |
| 78 | 89 |
| 80 | 89 |
| 85 | 91 |
| 87 | 92 |
| 92 | 85 |
| 96 | 87 |
| Native collagen type I | 100 |

The hydroxylation degree obtained with two integrations containing a P4H expression cassette ranges from 77 to 94 %. For clone 16 (K. phaffii 451-3) (90 % hydroxylation) the integration of the second P4H cassette took place in the AOX-locus on Chromosome 4.

### Example 9: Composition of Syn6 Medium

### 1.1 Basic medium: Syn6-MES-shake flask

Volume: 1000 ml flask size: 1000 ml

| Substance | Molecular weight | Amount [g] | Volume [ml] | Final concentration [g/l] |
|---|---|---|---|---|
| KH₂PO₄ | 136.09 | 1 | - | 1 |
| (NH₄)₂S0₄ | 132.14 | 7.66 | - | 7.66 |
| MES (200mM) | 195.2 | 39 | - | 39 |
| MgSO₄ x 7 H₂O | 246.48 | 3.0 | - | 3.0 |
| KCl | 74.56 | 3.3 | - | 3.3 |
| NaCl | 58.44 | 0.33 | - | 0.33 |
| deionized water | 18 | - | ad 940 | |
| Tri-Na citrate dihydrate | 294.1 | 3.42 | - | 3.42 |
| The pH-Value is set to 6.0 using 1M NaOH | | | | |

| Additives after autoclaving | | | | Final concentration [g/l] |
|---|---|---|---|---|
| Solution 1.1.1 (Calcium chloride) | | - | 10 | |
| Solution 1.1.2 (Microelements) | | - | 10 | |
| Solution 1.1.3 (Vitamins) | | - | 10 | |
| Solution 1.1.4 (Trace elements) | | - | 10 | |
| Solution 1.1.5 (Glucose) | | - | 20 | 10 |

### Solution 1.1.1 Calcium chloride 100 x stock

| Substance | Molecular weight | Amount [g] | Volume [ml] | Final concentration [g/l] |
|---|---|---|---|---|
| Calcium chloride x 2 H₂O | 147.02 | 10 | - | 100 |
| Deionized water | 18 | - | ad 100 | |

### Solution 1.1.2 Microelements 100 x stock

| Substance | Molecular weight | Amount [g] | Volume [ml] | Final concentration [g/l] |
|---|---|---|---|---|
| Titriplex III (EDTA) | 372.24 | 0.665 | - | 6.65 |
| Ammonium iron(II) sulphate x 6 H₂O | 392.14 | 0.665 | - | 6.65 |
| Copper(II) sulphate x 5 H₂O | 249.68 | 0.055 | - | 0.55 |
| Zinc(II) sulphate x 7 H₂O | 287.54 | 0.2 | - | 2.0 |
| Manganese(II) sulphate x H₂O | 169.02 | 0.265 | - | 2.65 |
| Deionized water | 18 | - | ad 100 | |

At first Titriplex III (EDTA) is weighted and dissolved in approximately 50 ml deionized water, then the further components are weighted and slowly dissolved in the total volume.

### Solution 1.1.3 Vitamins 100 x stock

Volume: 100 ml flask size: 100 ml

| Substance | Molecular weight | Amount [g] | Volume [ml] | Final concentration [g/l] |
|---|---|---|---|---|
| d-Biotin | 244.31 | 0.004 | - | 0.04 |
| 2-propanol/ELIX water (1:1) | | - | ad 10 | |
| Thiamine chloride | 337.27 | 1.335 | - | 13.35 |
| Deionized water | | - | ad 90 | |

d-Biotin is weighted as the first component and dissolved in 10 ml of a mixture of water and isopropanol (1:1). The thiamine chloride is weighted in a second flask and dissolved in the stated Amount of Elix water. Finally, both solutions are mixed.

### Solution 1.1.4 Trace elements 100 x stock

Volume: 100 ml flask size: 100 ml

| Substance | Molecular weight | Amount [g] | Volume [ml] | Final concentration [g/l] |
|---|---|---|---|---|
| Nickel(II) sulphate x 6 H₂O | 262.86 | 0.0065 | - | 0.065 |
| Cobalt(II) chloride x 6 H₂O | 237.93 | 0.0065 | - | 0.065 |
| Boric acid | 61.83 | 0.0065 | - | 0.065 |
| Potassium iodide x 7 H₂O | 166.01 | 0.0065 | - | 0.065 |
| Sodium molybdate x 2 H₂O | 241.95 | 0.0065 | - | 0.065 |
| Deionized water | 18 | - | ad 100 | |

### Solution 1.1.5 Glucose 500 g/L stock

Volume: 100 ml flask size: 100 ml

| Substance | Molecular weight | Amount [g] | Volume [ml] | Final concentration [g/l] |
|---|---|---|---|---|
| Glucose | 180.16 | 50 | - | 500 |
| Deionized water | 18 | - | ad 100 | |

### Sterilisation of solutions:

The solution 1.1.1 and solution 1.1.5 are heat sterilized at 121 °C for 20 min, whereas the remaining solutions 1.1.2, 1.1.3 and 1.1.4 are passed through a filter.

### Literature:

1. Lin-Cereghino J, Wong WW, Xiong S, Giang W, Luong LT, Vu J, Johnson SD, Lin-Cereghino GP. 2005.. Biotechniques 38:44, 46, 48.
2. Naatsaari L, Mistlberger B, Ruth C, Hajek T, Hartner FS, Glieder A. 2012. PLoS One 7:e39720.
3. Küberl A, Schneider J, Thallinger GG, Anderl I, Wibberg D, Hajek T, Jaenicke S, Brinkrolf K, Goesmann A, Szczepanowski R, Pühler A, Schwab H, Glieder A, Pichler H. 2011. J Biotechnol 154:312-320.
4. Sturmberger L, Chappell T, Geier M, Krainer F, Day KJ, Vide U, Trstenjak S, Schiefer A, Richardson T, Soriaga L, Damhofer B, Birner-Gruenberger R, Glick BS, Tolstorukov I, Cregg J, Madden K, Glieder A. 2016. J Biotechnol 235:121-131.
5. Stöckmann C, Maier U, Anderlei T, Knocke C, Gellissen G, Büchs J. 2003. J Ind Microbiol Biotechnol 30:613-622.
6. Scheidle M, Jeude M, Dittrich B, Denter S, Kensy F, Suckow M, Klee D, Büchs J. 2010. H FEMS Yeast Res 10:83-92.
7. Bähr C, Leuchtle B, Lehmann C, Becker J, Jeude M, Peinemann F, Arbter R, Büchs J. 2012. Biochemical Engineering Journal 69:182-195.
8. Philip P, Meier K, Kern D, Goldmanns J, Stockmeier F, Bahr C, Buchs J. 2017. Microb Cell Fact 16:122.
9. Anderlei T, Büchs J. 2001. Biochem Eng J 7:157-162.
10. Habicher T, John A, Scholl N, Daub A, Klein T, Philip P, Biichs J. 2019. Biotechnol Bioeng 116:1326-1340.
11. Lussier M, Sdicu AM, Camirand A, Bussey H. 1996. J Biol Chem 271:11001-11008.
12. Lussier M, Sdicu AM, Bussey H. 1999. Biochim Biophys Acta 1426:323-334.
13. Rutschmann C, Baumann S, Cabalzar J, Luther KB, Hennet T. 2014. Appl Microbiol Biotechnol 98:4445-4455.
14. Barrero JJ, Casler JC, Valero F, Ferrer P, Glick BS. 2018. Microbial Cell Factories 17:161.
15. Schmitz A, Ebert BE, Blank LM. 2017. p 223-243. In McGenity TJ, Timmis KN, Nogales B (ed), Hydrocarbon and Lipid Microbiology Protocols: Genetic, Genomic and System Analyses of Pure Cultures doi:10.1007/8623_2015_78. Springer Berlin Heidelberg, Berlin, Heidelberg.

## Claims

1. A fungal cell, which fungal cell comprises at least one copy of an exogenous nucleic acid sequence of interest stably integrated in yurl gene of the fungal cell, which exogenous nucleic acid sequence of interest encodes a recombinant peptide of interest.

2. The fungal cell of claim 1, wherein the fungal cell comprises in addition at least one copy, preferably two copies, of an exogenous nucleic acid sequence encoding a P4H (Prolyl-4-hydroxylase), a PIN4H (Proline-4-hydroxylase), a lysyl hydroxylase or two of these or all three, which at least one copy is stably integrated into at least one chromosome of the fungal cell, preferably not in the yurl gene.

3. The fungal cell according to any one of the preceding claims, which comprises at least three, in particular at least five, preferably 3, 4, 5 or 6, copies of an exogenous nucleic acid sequence of interest, preferably in the same orientation on the chromosome.

4. The fungal cell according to any one of the preceding claims, wherein the at least one copy of the exogenous nucleic acid sequence of interest is stably integrated at the 3' end of the protein coding region of a functional yurl gene.

5. The fungal cell according to any one of the preceding claims, wherein the exogenous nucleic acid sequence of interest encoding a recombinant peptide of interest is encoding a collagen peptide, in particular a type I collagen peptide, in particular having the nucleic acid sequence of SEQ ID No. 1 or a functionally equivalent sequence variant thereof, the variant having at least 80% sequence identity to SEQ ID No 1 or the variant being capable of hybridizing under low, medium or high stringency conditions with the nucleic acid sequence of SEQ ID No. 1 or its complementary strand.

6. The fungal cell according to any one of the preceding claims, which comprises at least three, preferably at least five, copies of the exogenous nucleic acid sequence of interest encoding the peptide of interest and one or two copies of an exogenous nucleic acid sequence encoding a P4H (Prolyl-4-hydroxylase), a PIN4H (Proline-4-hydroxylase), a lysyl hydroxylase or two of these or all three, wherein the peptide of interest is a collagen peptide.

7. The fungal cell according to any one of the preceding claims, wherein the fungal cell is selected from the group consisting of a Candida cell, Hansenula cell, Torulopsis cell, Kluyveromyces cell, Cyberlindnera cell, Rhodotorula cell, Yarrowia cell, Lipomyces cell and Komagataella cell, in particular Komagataella phaffii cell.

8. The fungal cell according to any one of the preceding claims, wherein the recombinant peptide of interest encoded by the exogenous nucleic acid sequence of interest is a fusion protein, in particular comprising a collagen peptide, which is in N- or/and C-terminal direction fused to one or more functional peptides.

9. The fungal cell according to any one of the preceding claims, wherein the recombinant peptide of interest encoded by the nucleic acid sequence of interest is a fusion protein comprising as a first element, as seen from the N-terminus, a functional peptide, in particular a secretion signal, in particular from mating factor α from Saccharomyces cerevisiae, and as a second element a collagen peptide and, optionally, as a third element a further functional peptide.

10. The fungal cell according to any one of the preceding claims, wherein the exogenous nucleic acid sequence of interest comprises a nucleic acid sequence encoding the recombinant peptide of interest and at least one regulatory unit, in particular a promoter, an enhancer, a silencer and/or a terminator.

11. The fungal cell according to any one of the preceding claims, wherein the at least one copy of the exogenous nucleic acid sequence of interest is integrated in a yurl gene, which yurl gene is in a non-native position on the chromosome, in particular chromosome 2, of the fungal cell, preferably Komagataella phaffii.

12. The fungal cell according to claim 11, wherein the non-native position of the yurl gene is a central position on the chromosome, preferably located in the 3'-direction of the native position of the yurl gene on chromosome 2 of Komagataella phaffii.

13. A fungal cell, which is a Komagataella phaffii cell, preferably according to any one of the preceding claims, as deposited with the DSMZ under accession numbers: Komagataella phaffii 451-1 (DSM 33955, deposited on 28.07.2021), Komagataella phaffii 451-2 (DSM 33956, deposited on 28.07.2021), Komagataella phaffii 451-3 (DSM 33957, deposited on 28.07.2021), a further derivate of Komagataella phaffii 451-1, Komagataella phaffii 451-4 (DSM 33958, deposited on 28.07.2021) or a derivate of Komagataella phaffii 451-4.

14. An expression vector comprising an expression cassette, which expression cassette comprises at least one nucleic acid sequence of interest, in particular encoding a collagen peptide,
and at least one nucleic acid sequence of at least 10, preferably at least 25, preferably at least 50, preferably at least 100, preferably at least 150, preferably all, nucleotides of the yurl gen, preferably as identified in SEQ ID No. 13.

15. A host cell containing the expression vector of claim 14.

16. A process for producing a fungal cell according to any one of the preceding claims, which comprises:
x) providing a fungal host cell and an expression vector comprising at least one expression cassette, which expression cassette comprises at least one exogenous nucleic acid sequence of interest,
y) transforming the fungal host cell with the expression vector under appropriate conditions so as to effect integration of the at least one copy of an exogenous nucleic acid sequence stably into the yurl gene, and
z) obtaining the fungal cell.

17. A process for producing a recombinant peptide of interest in a fungal cell, which process comprises the steps of
a) providing a fungal cell according to any one of claims 1 to 13 or obtained according to the method of claim 16,
b) culturing the fungal cell under conditions suitable for the expression of the recombinant peptide of interest, and
c) obtaining the recombinant peptide of interest.

18. The process of claim 17 for producing a recombinant peptide of interest, which is a process for producing a hydroxylated recombinant peptide of interest in a fungal cell, and comprises the steps of
ax) providing a fungal cell according to any one of claims 1 to 13, which comprises at least one copy of an exogenous nucleic acid sequence of interest stably integrated into the yurl gene and at least one copy of an exogenous nucleic acid sequence encoding a P4H, PIN4H or lysyl hydroxylase, in particular a P4H, and, optionally, a culture medium,
bx) culturing the fungal cell under conditions suitable for the expression and hydroxylation of the recombinant peptide of interest, and
cx) obtaining the recombinant hydroxylated recombinant peptide of interest.

19. A recombinant peptide of interest obtainable by a process according to claim 17 or 18.

20. A cell culture comprising a fungal cell of any one of claims 1 to 13, optionally in combination with a culture medium.

21. A bioreactor comprising the cell culture of claim 20.
